# EUROPÄISCHE PATENTSCHRIFT

(11) **EP 0 805 152 B1**
(45) Veröffentlichungstag und Bekanntmachung des Hinweises auf die Patenterteilung: **08.06.2005**
(21) Anmeldenummer: 97810255.6
(22) Anmeldetag: 24.04.1997
(51) Int. Cl.: C07D 295/10, C07D 295/12, C07D 265/28, C07D 241/04, C07D 211/14

(54) **Cyclisches Amin substituiertes Phenyl-alkyl-Keton und Verfahren zu dessen Herstellung**
Cyclic amine substituted phenyl-alkyl-ketone and process for its preparation
Phenyl-alkyl-cétone substitué avec une amine cyclique et procédé pour sa préparation

(30) Priorität: 03.05.1996 CH 113896
(43) Veröffentlichungstag der Anmeldung: 05.11.1997
(60) Teilanmeldung: 04102145.2
(73) Patentinhaber: Ciba Specialty Chemicals Holding Inc., 4057 Basel (CH)
(72) Erfinder: Hüsler, Rinaldo, 3184 Wünnewil (CH); Schwabe, Rudolf, 3076 Worb (CH); Luisoli, Reto, 4434 Hölstein (CH)

(56) Entgegenhaltungen:
- EP-A- 0 138 754
- EP-A- 0 284 561
- EP-A- 0 378 207
- EP-A- 0 401 715
- GB-A- 1 164 608
- US-A- 3 317 538
- CHEMICAL ABSTRACTS, vol. 108, no. 10, 7.März 1988 Columbus, Ohio, US; abstract no. 85724k, BARTULIN ET AL.: "Synthesis and mesogenic properties of ..." XP002038120
- ISHIHARA ET AL.: "Central cholinergic agents ..." CHEM.PHARM.BULL., Bd. 41, Nr. 3, 1993, Seiten 529-538, XP002038119

## Beschreibung

Die Erfindung betrifft neue, mit cyclischem Amin substituierte Phenyl-alkyl-Ketone und ihre Verwendung zur Herstellung von Photoinitiatoren für die Photopolymerisation von ethylenisch ungesättigten Verbindungen.

In der EP-B-0284 561 sind α-Aminoacetophenone offenbart, welche als Photoinitiatoren verwendet werden. Hergestellt werden diese Verbindungen durch eine Reihe von Verfahrensschritten, welche im Falle von aromatischen Aminen immer von einem Derivat eines p-Fluorphenylalkylen-1-on ausgehen, wobei in der letzten Synthesestufe das p-ständige Fluor durch eine Aminogruppe ausgetauscht wird. Dieser Austausch erfolgt in einem organischen Lösungsmittel, z.B. Dimethylformamid oder Dimethylsulfoxid, in Gegenwart von Kaliumcarbonat.

Aufgabe der Erfindung war es u.a. einerseits Reaktionen zu entwickeln, welche Fluoraromaten vermeiden, da diese ökologisch problematisch sind, eine unerwünschte Beseitigung der Abfälle verursachen und gegenüber Aminen, wegen ihrer vergleichsweisen hohen Reaktivität, empfindlich sind, und andererseits von organischen Lösungsmitteln wegzukommen, da ein Arbeiten in diesen zu mehr oder weniger dunkel gefärbten Produkten mit Nebenprodukten führt, d.h. einerseits weniger reine Produke und geringere Ausbeuten erhalten werden.

Die aus der Literatur bekannten Verfahren, wonach in einem Alkylphenylketon mit p-ständigem Halogen, vor allem Fluor oder Chlor, im Phenylkern das Halogen gegen einen Aminrest ausgetauscht wird, arbeiten:
a) in einem organischen Lösungsmittel (z.B. EP-B-0138754, Umsetzung von 1-(4-fluorphenyl)-2-methyl-propanon-1 mit Piperidin in Dimethylsulfoxid; CH 200 365, Umsetzung von p-Chlorstearophenon mit Dimethylamin in Ethanol in Gegenwart von Kupferpulver als Katalysator; T. lbata, Y. lsogami, J. Toyoda, Bull. Chem. Soc. Jpn. 64(1)42-49 (1991), Umsetzung von Chloracetophenon mit Pyrrolidin in Tetrahydrofuran unter Anwendung extrem hoher Drucke (7,2 Kbar); J. Org. Chem. 31(7), 2319-21 (1966), Umsetzung von 1-(4-Fluorphenyl)-propan-1-on mit alicyclischen Aminen, wie Piperidin, in Dimethylformamid oder Dimethylsulfoxid, oder
b) ohne Lösungsmittel (z.B. G. Kresze und H. Goetz, Chem. Berichte 90, 2161, 2174 (1957)), Umsetzung von p-Bromacetophenon mit Piperidin unter Rückfluss mit Ausbeuten an 1-(4-piperidinophenyl)-ethanon von 19 %; oder
c) in Wasser (z.B.: T. Lundstedt, P. Thoren, R. Carlson, Acta Chemica Scand. B 38, 1984 No. 8 S. 717-719; Umsetzung von p-Chloracetophenon mit Dimethylamin unter Druck in Wasser; US-A-1'946'058, Umsetzung von p-Chloracetophenon mit wässrigem Ammoniak in Wasser unter Druck in Gegenwart von Kupferoxid als Katalysator; JP 78-40404, Umsetzung von p-Chloracetophenon mit Mono- oder Dialkylaminen in Wasser unter Druck in Gegenwart von Kupferpulver als Katalysator) wo einerseits Explosionen auftraten und andererseits die Ausbeuten unter 80 % liegen.

Phenyl-alkyl-ketone, welche in p-Stellung im Phenylkern durch ein cyclisches Amin substituiert sind, und zudem in α-Stellung zur Ketogruppe eine freie Methylengruppe besitzen sind nur wenig bekannt; verwiesen wird z.B. auf die EP-B-0'138'754 (2-Methyl-1(4-piperidinophenyl)-propanon-1); CH 200365 (p-Dimethylaminostearophon, wobei anstelle des Dimethylaminorestes gemäss Beschreibung auch Piperidin möglich ist, ohne aber ein konkretes Beispiel zu geben); G. Kresze und H. Goetz, Chem. Berichte 90, 2161, 2174 (1957), (1-(4-Piperidinophenyl)-ethanon); T. lbata, Y. Isogami, J. Toyoda, Bull. Chem. Soc. JPn. 64(1), 42-49 (1991), (1-(4-Pyrrolidon)acetophenon); und J. Org. Chem. 31(7), 2319-21 (1966), (1-(4-Piperidinophenyl)-propanon-1).

Die Erfindung, und gleichzeitig die Lösung der gestellten Aufgabe, betrifft neue mit cyclischem Amin p-substituierte Phenyl-alkyl-ketone, welche u.a. als neue Zwischenprodukte zur Herstellung von bestimmten Photoinitiatoren verwendet werden können

Bei den neuen, erfindungsgemässen, mit cyclischem Amin p-substituierten Phenyl-alkyl-Ketonen handelt es sich um die Verbindungen
1-(4-morpholinophenyl)butan-1-one, 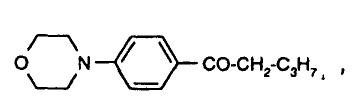 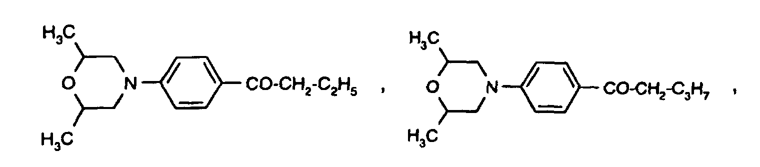 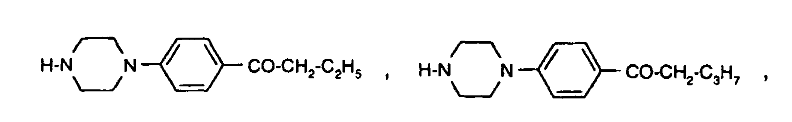 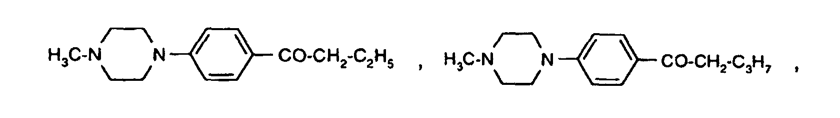 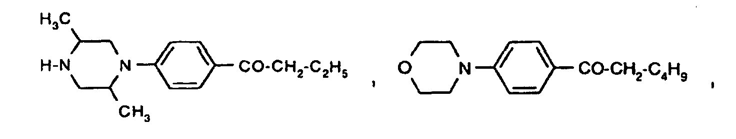 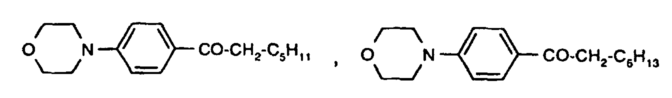 und

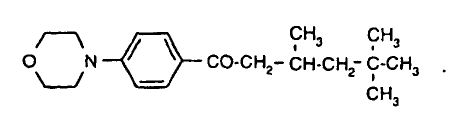

Die Herstellung der erfindungsgemässen Verbindungen erfolgt nach einem neuen Verfahren:

Dieses Verfahren zur Herstellung von Verbindungen der Formel I 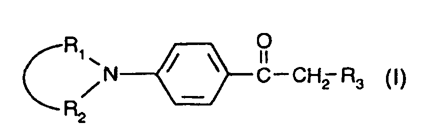 worin bedeuten:
R₁ und R₂ zusammen unter Einschluss des N-Atoms einen Morpholinyl, Dimethylmorpholinyl-, Piperazinyl-, N-Methylpiperazinyl- oder 2,5-Dimethylpiperazinylrest; und
R₃ unverzweigtes oder verzweigtes, unsubstituiertes oder substituiertes C₂-C₂₀-Alkyl; besteht in der Aminolyse eines p-Halogenphenylalkylketons der Formel II 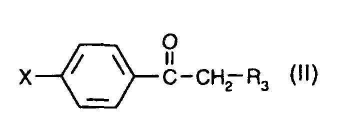
mit einem cyclischen Amin der Formel III 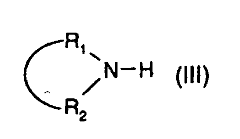 in Wasser bei einer Temperatur von mindestens 130°C in welchen Formeln X ein Halogenatom bedeutet und R₁, R₂ und R₃ die unter Formel I angegebene Bedeutung haben.

Vorzugsweise wird als p-Halogenphenylalkylketon der Formel II ein solches verwendet worin X Brom und insbesondere Chlor bedeutet.

Bedeutet R₃ einen unsubstituierten oder substituierten C₂-C₂₀-Alkylrest, so kann auch dieser unverzweigt oder verzweigt sein. Beispielsweise handelt es sich um folgende Alkylreste:
Ethyl, Propyl, n-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Isopropyl, sec-Butyl, Isobutyl, tert-Butyl, 2-Ethylbutyl, Isopentyl, 1-Methylpentyl, 1,3-Dimethylbutyl, 1-Methylhexyl, Isoheptyl, 1,1,3,3-Tetramethylbutyl, 2,2,4,4-Tetramethylbutyl, 1-Methylheptyl, 3-Methylheptyl, 2-Ethylhexyl, 1,1,3-Trimethylhexyl, 1,1,3,3-Tetramethylpentyl, Isodecyl, 1-Methylundecyl oder 1,1,3,3,5,5-Hexamethylhexyl.

Diese C₂-C₂₀-Alkylreste können ferner noch ein- oder mehrmals substituiert sein, z.B. durch Cyclohexyl, Phenyl, C₁-C₄-Alkoxy oder Phenoxy.

Es handelt sich dann beispielsweise um die R₃-Reste: 2-Methoxyethyl, 3-Butoxypropyl, 2-Isopropoxyethyl, 4-Phenoxybutyl, 2-Phenylethyl oder 3-Phenylpropyl.

Besonders bevorzugte R₃ Alkylreste sind unsubstituierte, unverzweigte oder verzweigte Alkylreste mit 2 bis 10 Kohlenstoffatomen, vor allem solche mit 2 bis 7 Kohlenstoffatomen, insbesondere mit 2 bis 5 Kohlenstoffatomen wie Ethyl oder Propyl.

Vorteilhaft ist die Menge an cyclischem Amin der Formel III im Ueberschuss, bezogen auf das p-Halogenphenylalkylketon der Formel II, vorhanden. Dieser Ueberschuss beträgt vor allem etwa 2,5-20 insbesondere 2,5-12 Moläquivalente.

Die Menge an Wasser liegt zwischen etwa 1 bis 100 vorzugsweise 2 bis 20 und insbesondere 2,5 bis 10 Moläquivalenten bezogen auf 1 Moläquivalent p-Halogenphenylalkylketon der Formel II; grössere Wassermengen sind aber auch nicht kritisch.

Die Umsetzung wird vorteilhafterweise unter Druck (ca. 3-30 bar) in einem Druckkessel, vor allem in einem Hochdruckreaktor aus Stahl mit Flügelrührer, Manometer und Thermoelement durchgeführt. Es ist aber auch möglich die Reaktion ohne Druckkessel unter Rückfluss (ca. 105°C - 110°C) durchzuführen.

Zweckmässig liegt die Temperatur in einem Temperaturbereich von etwa 140°C bis 240°C insbesondere 150°C bis 230°C. Arbeitet man mit dem p-Bromphenylalkylketon der Formel II so liegt der Temperaturbereich zwischen etwa 140°C und 200°C vorteilhaft zwischen 160°C - 180°C, und arbeitet man mit dem p-Chlorphenylalkylketon der Formel II so liegt der Temperaturbereich zwischen etwa 180°C und 240°C, vorteilhaft zwischen 200°C und 230°C.

Katalysatoren können, müssen aber nicht zugesetzt werden; diese beschleunigen die Reaktion zwar in einem gewissen Ausmass, aber andererseits vermindert ein Arbeiten ohne Katalysator ökologische Probleme und relativisiert die Vorteile eines Schwermetallzusatzes.

Als Katalysatoren kommen vor allem in Betracht:
Kupfer- oder Nickelverbindungen oder deren Salze, wie Kupfer(I)chlorid, Kupfer(I)bromid, Kupfer(I)jodid, Kupfer(ll)bromid, Kupfer(ll)chlorid, Kupfercarbonat, Kupfer(ll)sulfat, Kupferoxid sowie Kupferpulver, oder Nickelacetat, Nickeloxid, Nickelchlorid und Nickelbromid.

Diese Katalysatoren werden in Mengen von etwa 0,1-15 Gew.%, insbesondere 0,5 - 5 Gew.% bezogen auf 100,0 Gew.% p-Halogenphenylalkylketon der Formel II zugesetzt.

Weitere Lösungsmittel sind im Prinzip zur Durchführung der Umsetzung nicht nötig, können aber mitverwendet werden; zweckmässig haben sich dabei hochsiedende und polare Lösungsmittel bewährt wie Diethylenglykol, Diethylenglykolmonomethylether, Diethylenglykoldimethylether, Benzylalkohol, Phenylethylalkohol oder Phenoxyethanol.

Die Umsetzung des cyclischen Amins der Formel III mit dem p-Halogenphenylalkylketon der Formel II wird vorteilhafterweise derart durchgeführt, dass entweder:
a) das p-Halogenphenylalkylketon der Formel II im Reaktionsgefäss zusammen mit dem Wasser und dem cyclischen Amin vorgelegt wird und sofort bis zur Endtemperatur aufgeheizt wird, oder
b) das p-Halogenphenylalkylketon der Formel II wird im Reaktionsgefäss zusammen mit dem Wasser und dem Amin vorgelegt und langsam über Stunden während der Reaktion bis zur Endtemperatur aufgeheizt, oder
c) das p-Halogenphenylalkylketon der Formel II wird während der Reaktion, zweckmässig geschmolzen, zum Wasser und dem cyclischen Amin, das vorgängig auf die Reaktionstemperatur aufgeheizt wurde, zudosiert. Diese Verfahrensvariante reduziert oder eliminiert vor allem das Risiko einer autokatalytischen Zersetzung bei sehr hohen Temperaturen. Beispielsweise kann so verfahren werden, dass alle Komponenten im Reaktionsgefäss vorgelegt werden und das p-Bromphenylalkylketon der Formel II in einem Temperaturbereich von etwa 140°C-190°C während Stunden zudosiert wird, wobei die Temperatur während etwa 3-12 Stunden vom tieferen Niveau allmählich aufs höhere Niveau erhöht wird, oder, dass das p-Chlorphenylalkylketon der Formel II in einem Temperaturbereich von etwa 180°C-230°C während Stunden zudosiert wird, wobei die Temperatur während etwa 3-12 Stunden vom tieferen Niveau allmählich aufs höhere Niveau erhöht wird.

Aus Sicherheitsgründen ist es zweckmässig die Akkumulation des p-Halogenphenylalkylketons unter Kontrolle zu halten.

Bevorzugte Verfahrens-Arbeitsweisen bestehen z.B. darin, dass 1 Teil (Teile sind hier und im folgenden auf Molmengen bezogen) p-Bromphenylalkylketon bzw. 1 Teil p-Chlor-phenylalkylketon der Formel II worin R₃ unverzweigtes oder verzweigtes, unsubstituiertes C₂-C₇-Alkyl bedeutet mit 5 Teilen eines cyclischen Amins der Formel III worin R₁ und R₂ zusammen unter Einschluss des N-Atoms einen Morpholinyl, Dimethylmorpholinyl-, Piperazinyl-, N-Methylpiperazinyl- oder 2,5-Dimethylpiperazinylrest bedeuten, und 5 Teile Wasser im Reaktionsgefäss vorgelegt werden und unter Druck bei einer Temperatur von etwa 160°C - 180°C bzw. 200°C - 230°C umgesetzt werden oder, dass 10 bis 20 Teile eines cyclischen Amins der Formel III, worin R₁ und R₂ zusammen unter Einschluss des N-Atoms einen Morholinyl, Dimethylmorpholinyl-, Piperazinyl-, N-Methylpiperazinyl- oder 2,5-Dimethylpiperazinylrest bedeuten, und 20 bis 40 Teile Wasser im Reaktionsgefäss vorgelegt werden, 2 bis 4 Teile p-Chlorphenylalkylketon der Formel II, worin R₃ unverzweigtes oder verzweigtes, unsubstituiertes C₂-C₇-Alkyl bedeutet zudosiert werden und unter Druck bei etwa 210°C - 230°C umgesetzt werden.

Die Aufarbeitung und Reinigung der neuen Phenyl-alkyl-Ketone der Formel I, welche mit einem cyclischen Amin substituiert sind, erfolgt nach bekannten Methoden, z.B. mittels Destillation, Kristallisation und Filtration.

Die cyclischen Amine der Formel III sind bekannt, teilweise im Handel erhältlich, und können nach bekannter Art und Weise hergestellt werden (z.B. Houben-Weyl, Vol. 11/1 (1957) S. 26-29, 32-33 und 63-67; Org. Synth. Coll. Vol. 3, 307 (1955); JACS 109, 1496-1502 (1987) oder Tetrahedron Vol. 40, 1433-1456 (1984).

Beispielsweise handelt es sich um folgende Verbindungen: Morpholin, Piperazin, N-Methylpiperazin, 2,6-Dimethylmorpholin oder Dimethylpiperazin.

Die p-Halogenphenylalkylketone der Formel II sind ebenfalls bekannt (z.B. Friedel-Crafts and related Reactions, Ed. C.A. Olah, J. Wiley and Sons, N.Y. (1964) Vol. 3, Parts 1+2; Chem. Rev. 55, 229 (1955); Org. Synth. Coll. Vol. 3,14 (1955) und JACS 109,7122 (1987).

Beispiele für einzelne Verbindungen sind: 1-(4-Bromphenyl)-n-butan-1-on, -n-pentan-1-on, -n-hexan-1-on, -n-heptan-1-on, -n-octan-1-on, -iso-nonan-1-on, 1-(4-Chlorphenyl)-n-butan-1-on und 1-(4-Chlorphenyl)-n-pentan-1-on.

Die Herstellung der p-Halogenphenylalkylketone der Formel II erfolgt nach bekannter Art und Weise z.B. mittels einer Friedel-Crafts-Reaktion aus einem Halogenbenzol und einem Alkancarbonsäurechlorid nach folgendem Reaktionsschema:

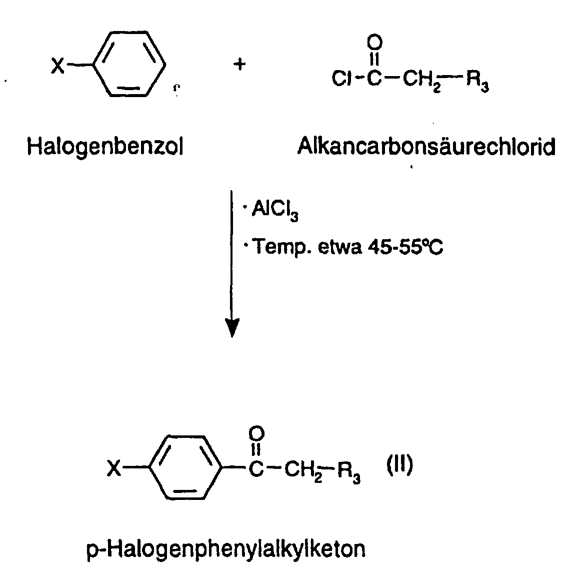

In diesen Formeln bedeutet X ein Halogenatom, vorzugsweise Chlor oder Brom, und R₃ hat die weiter vorne angegebene Bedeutung.

Beide Edukte, das Halogenbenzol und das Alkancarbonsäurechlorid, sind bekannt.

Beispiele für Halogenbenzole sind vor allem das Monobrombenzol und insbesondere das Monochlorbenzol.

Beispiele für Alkancarbonsäurechloride sind z.B. Buttersäurechlorid, lsobuttersäurechlorid, n-Valeriansäurechlorid, Isovaleriansäurechlorid, Capronsäurechlorid, Oenanthsäurechlorid, Caprylsäurechlorid, Pelargonsäurechlorid, Caprinsäurechlorid, Laurinsäurechlorid, Myristinsäurechlorid, Palmitinsäurechlorid, Stearinsäurechlorid, Arachinsäurechlorid, Eikosancarbonsäurechlorid und Behensäurechlorid.

Das gemäss der Umsetzung erhaltene p-Halogenphenylalkylketon der Formel II muss vor dessen weiteren Umsetzung mit dem cyclischen Amin der Formel III isoliert werden.

Es ist überraschend, und war aus der eingangs genannten Literatur nicht vorhersehbar, dass der Zusatz von Wasser zur Reaktion des p-Halogenphenylalkylketons der Formel II mit dem cyclischen Amin der Formel III sehr effizient die Bildung von farbigen Nebenprodukten und Verharzungen verhindert, und sehr reine, helle Produkte, die eine Reinheit von >99,0 % besitzen, erhalten werden.

Zudem bringt ein Arbeiten in Wasser, vor allem grosstechnisch, im Vergleich zu organischen Lösungsmitteln, wie Dimethylsulfoxid oder Dimethylformamid, ökologische Vorteile mit sich.

Es ist weiterhin überraschend, dass die Reaktion des p-Halogenphenylalkylketons der Formel II mit dem cyclischen Amin der Formel III, d.h. der Halogen-Austausch bei einem wenig aktivierten Benzolderivat so glatt und so schnell in Wasser verläuft.

Ferner ist es auch überraschend, dass die Aminolyse-Reaktion ohne zwingenden Zusatz eines Katalysators verläuft und hohe Ausbeuten von 88 % bis 96 % erzielt werden; durch die Abwesenheit eines Katalysators erspart man sich auch die Entfernung desselben aus dem Endprodukt, welches meistens ein zeitaufwendiges Verfahren ist.

Verwendet werden die Verbindungen der Formel I 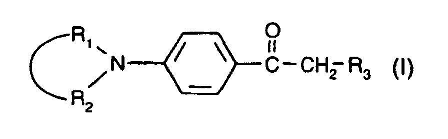 worin bedeuten:
R₁ und R₂ zusammen unter Einschluss des N-Atoms einen Morpholinyl, Dimethylmorpholinyl-, Piperazinyl-, N-Methylpiperazinyl- oder 2,5-Dimethylpiperazinylrest;
R₃ unverzweigtes oder verzweigtes, unsubstituiertes oder substituiertes C₂-C₂₀-Alkyl,
vor allem zur Herstellung von radikalischen Photoinitiatoren der Formel IV 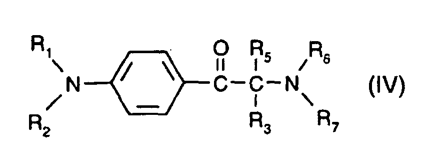 oder deren Säureadditionssalzen, worin bedeuten:
R₁ und R₂ zusammen unter Einschluss des N-Atoms einen Morpholinyl, Dimethylmorpholinyl-, Piperazinyl-, N-Methylpiperazinyl- oder 2,5-Dimethylpiperazinylrest;
R₃ unverzweigtes oder verzweigtes, unsubstituiertes oder durch C₁-C₄-Alkoxy, Phenoxy, Cyclohexyl oder Phenyl substituiertes C₂-C₂₀-Alkyl,
R₅ entweder
   (a) ein Rest der Formel 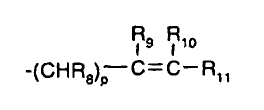 worin p null oder 1 ist,
      oder
   (b) ein Rest der Formel 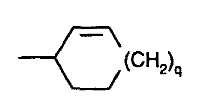 ist, wobei q 0, 1, 2 oder 3 bedeutet
      oder
   c) ein Rest der Formel 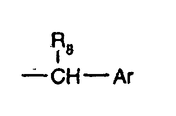
worin Ar einen unsubstituierten oder durch Halogen, OH, C₁-C₁₂-Alkyl oder durch OH, Halogen, -N(R₁₂)₂, -C₁-C₁₂-Alkoxy, -COO(C₁-C₁₈-Alkyl), -CO(OCH₂CH₂)ₙOCH₃ oder -OCO(C₁-C₄)-Alkyl substituiertes C₁-C₄-Alkyl; C₁-C₁₂-Alkoxy, oder durch -COO(C₁-C₁₈-Alkyl) oder -CO(OCH₂CH₂)ₙOCH₃ substituiertes C₁-C₄-Alkoxy; -(OCH₂CH₂)ₙOH, -(OCH₂CH₂)ₙOCH₃, C₁-C₈-Alkylthio, Phenoxy, -COO(C₁-C₁₈-Alkyl), -CO(OCH₂CH₂)ₙOCH₃, Phenyl oder Benzoyl substituierten Phenyl-, Naphthyl-, Furyl-, Thienyl- oder Pyridylrest bedeutet, worin n 1 - 20 ist,
in welchen Formeln
R₁₂ Wasserstoff, C₁-C₈-Alkyl, C₃-C₅-Alkenyl, C₇-C₉-Phenylalkyl, C₁-C₄-Hydroxyalkyl oder Phenyl bedeutet,
R₈ Wasserstoff, C₁-C₈-Alkyl oder Phenyl bedeutet, und
R₉, R₁₀ und R₁₁ unabhängig voneinander Wasserstoff oder C₁-C₄-Alkyl bedeuten oder R₉ und R₁₀ zusammen C₃-C₇-Alkylen sind,
R₆ Wasserstoff, C₁-C₁₂-Alkyl, durch Hydroxy, C₁-C₄-Alkoxy, -CN oder -COO(C₁-C₄-Alkyl) substituiertes C₂-C₄-Alkyl; C₃-C₅-Alkenyl, C₅-C₁₂-Cycloalkyl oder C₇-C₉-Phenylalkyl bedeutet, R₇ C₁-C₁₂-Alkyl, durch Hydroxy, C₁-C₄-Alkoxy, -CN oder -COO(C₁-C₄-Alkyl) substituiertes C₂-C₄-Alkyl; C₃-C₅-Alkenyl, C₅-C₁₂-Cycloalkyl, C₇-C₉-Phenylalkyl, Phenyl oder durch Halogen, C₁-C₁₂-Alkyl, C₁-C₄-Alkoxy oder -COO(C₁-C₄-Alkyl) substituiertes Phenyl bedeutet oder R₇ zusammen mit R₃ C₁-C₇-Alkylen, C₇-C₁₀-Phenylalkylen, o-Xylylen, 2-Butenylen oder C₂-C₃-Oxa- oder Azaalkylen bedeutet, oder
R₆ und R₇ zusammen C₃-C₇-Alkylen bedeuten, das durch -O-, -S-, -CO- oder -N(R₁₃)-unterbrochen sein kann oder durch Hydroxy, C₁-C₄-Alkoxy oder -COO(C₁-C₄-Alkyl) substituiert sein kann, wobei R₁₃ Wasserstoff, C₁-C₁₂-Alkyl, das durch ein oder mehrere -O-unterbrochen sein kann, C₃-C₅-Alkenyl, C₇-C₉-Phenylalkyl, C₁-C₄-Hydroxyalkyl, -CH₂CH₂CN, -CH₂CH₂COO(C₁-C₄-Alkyl), C₂-C₈-Alkanoyl oder Benzoyl bedeutet.

Das erfindungsgemässe Verfahren erlaubt so auf einfache, grosstechnisch sehr gut realisierbare, Art und Weise die Herstellung von Photoinitiatoren der Formel IV ausgehend von Monohalogenbenzol und einem Säurechlorid der Formel 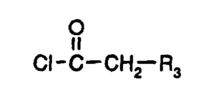 mittels einer Friedel-Crafts-Reaktion zu einem p-Halogenphenylalkyl-Keton der Formel II und dessen Aminolyse,
mit einem cyclischen Amin der Formel III 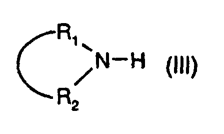 in Wasser bei einer Temperatur von mindestens 130°C in welchen Formeln X ein Halogenatom bedeutet und R₁, R₂ und R₃ die oben angegebene Bedeutung haben, zu einem cyclischen Amin substituierten Phenyl-alkyl-Keton der Formel I

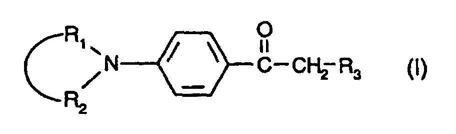

Halogenierung dieser Phenyl-alkyl-Keton-Verbindung der Formel I, Umsetzung mit einem Amin der Formel 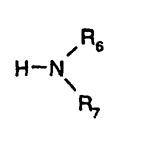 anschliessender Umsetzung mit einer den Rest R₅ einführenden Verbindung und Stevens-Umlagerung unter basischen Konditionen.

Bei der Halogenierung der Phenyl-alkyl-Keton Verbindung der Formel I handelt es sich um eine α-Halogenierung mit z.B. Brom oder Chlor in einem Lösungsmittel wie z.B. Eisessig bei Raumtemperatur. Die anschliessende Aminierung mit einem Amin der Formel 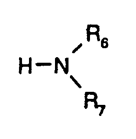 worin R₆ und R₇ die weiter oben angegebene Bedeutung haben (z.B. Dimethylamin) wird in einem geeigneten Lösungsmittel z.B. Methylethylketon durchgeführt. Anschliessend an die Aminierung erfolgt die Umsetzung mit einer die Gruppe R₅ einführenden Verbindung beispielsweise Benzylbromid, Benzylchlorid, Allylbromid oder Allylchlorid und daran anschliessend die Stevens-Umlagerung unter basischen Bedingungen, z.B. NaOH oder KOH.

Durch das Vorhandensein einer basischen Aminogruppe lassen sich die Photoinitiatoren der Formel IV durch Addition von Säuren in die entsprechenden Säureadditionssalze überführen. Die Säuren können anorganische oder organische Säuren sein. Beispiele für solche Säuren sind HCI, HBr, H₂SO₄, H₃PO₄, Mono- oder Polycarbonsäuren wie z.B. Essigsäure, Oelsäure, Bernsteinsäure, Sebacinsäure, Weinsäure oder CF₃COOH, Sulfonsäuren wie z.B. CH₃SO₃H, C₁₂H₂₅SO₃H, p-C₁₂H₂₅-C₆H₄-SO₃H, p-CH₃-C₆H₄-SO₃H oder CF₃SO₃H, Acrylsäure, Methacrylsäure, Polyacrylsäure, Polymethacrylsäure und Benzoesäure.

Photoinitiatoren für radikalisch polymerisierbare Verbindungen sind Verbindungen, welche bei Bestrahlung mit kurzwelligem Licht in radikalische Bruchstücke zerfallen, welche die eigentlichen Initiatoren für die Polymerisation der ethylenisch ungesättigten Verbindungen sind.

Diese Photoinitiatoren werden vor allem verwendet für die Photopolymerisation ethylenisch ungesättigter Verbindungen bzw. Gemischen, die solche Verbindungen enthalten, für die Photohärtung von pigmentierten Systemen wie Druckfarben oder Weisslacke, für die Photohärtung von unpigmentierten Systemen, wie UV-härtbare Druckfarben, für die Herstellung von Photoresists und Druckplatten und für Aussenanstriche, die im Tageslicht oberflächlich nachhärten.

Die ungesättigten Verbindungen können eine oder mehrere olefinische Doppelbindungen enthalten. Sie können niedermolekular (monomer) oder höhermolekular (oligomer) sein. Beispiele für Monomere mit einer Doppelbindung sind Alkyl- oder Hydroxyalkyl-acrylate oder -methacrylate, wie z.B. Methyl- , Ethyl-, Butyl-, 2-Ethylhexyl-oder 2-Hydroxyethylacrylat, Isobornylacrylat, Methyl- oder Ethylmethacrylat. Weitere Beispiele hierfür sind Acrylnitril, Acrylamid, Methacrylamid, N-substituierte (Meth)acrylamide, Vinylester wie Vinylacetate, Vinylether wie Isobutylvinylether, Styrol, Alkyl- und Halogenstyrole, N-Vinylpyrrolidon, Vinylchlorid oder Vinylidenchlorid.

Beispiele für Monomere mit mehreren Doppelbindungen sind Ethylenglykol-, Propylenglykol-, Neopentylglykol-, Hexamethylenglykol-, oder Bisphenol-A-diacrylat, 4,4'-Bis(2-acryloyloxyethoxy)-diphenylpropan, Trimethylolpropan-triacrylat, Pentaerythrittriacrylat oder -tetraacrylat, Vinylacrylat, Divinylbenzol, Divinylsuccinat, Diallylphthalat, Triallylphosphat, Triallylisocyanurat oder Tris-(2-acryloyloxyethyl)isocyanurat.

Beispiele für höhermolekulare (oligomere) mehrfach ungesättigte Verbindungen sind acrylierte Epoxidharze, acrylierte Polyether, acrylierte Polyurethane oder acrylierte Polyester. Weitere Beispiele für ungesättigte Oligomere sind ungesättigte Polyesterharze, die meist aus Maleinsäure, Phthalsäure und einem oder mehreren Diolen hergestellt werden und Molekulargewichte von etwa 500 bis 3000 besitzen. Solche ungesättigten Oligomere kann man auch als Prepolymere bezeichnen.

Häufig verwendet man Zweikomponenten-Gemische eines Prepolymeren mit einem mehrfach ungesättigten Monomeren oder Dreikomponentengemische, die ausserdem noch ein einfach ungesättigtes Monomer enthalten. Das Prepolymere ist hierbei in erster Linie für die Eigenschaften des Lackfilmes massgebend, durch seine Variation kann der Fachmann die Eigenschaften des gehärteten Filmes beeinflussen. Das mehrfach ungesättigte Monomere fungiert als Vernetzer, das den Lackfilm unlöslich macht. Das einfach ungesättigte Monomere fungiert als reaktiver Verdünner, mit dessen Hilfe die Viskosität herabgesetzt wird, ohne dass man ein Lösungsmittel verwenden muss.

Solche Zwei- und Dreikomponentensysteme auf der Basis eines Prepolymeren werden sowohl für Druckfarben als auch für Lacke, Photoresists oder andere photohärtbare Massen verwendet. Als Bindemittel für Druckfarben werden vielfach auch EinkomponentenSysteme auf der Basis photohärtbarer Prepolymerer verwendet.

Ungesättigte Polyesterharze werden meist in Zweikomponentensystemen zusammen mit einem einfach ungesättigten Monomer, vorzugsweise mit Styrol, verwendet. Für Photoresist werden oft spezifische Einkomponentensysteme verwendet, wie z.B. Polymaleinimide, Polychalkone oder Polyimide, wie sie in der DE-OS 2 308 830 beschrieben sind.

Die ungesättigten Verbindungen können auch im Gemisch mit nicht-photopolymerisierbaren filmbildenden Komponenten verwendet werden. Diese können z.B. physikalisch trocknende Polymere bzw. deren Lösungen in organischen Lösungsmitteln sein, wie z.B. Nitrocellulose oder Celluloseacetobutyrat. Diese können aber auch chemisch bzw. thermisch härtbare Harze sein, wie z.B. Polyisocyanate, Polyepoxide oder Melaminharze. Die Mitverwendung von thermisch härtbaren Harzen ist für die Verwendung in sogenannten Hybrid-Systemen von Bedeutung, die in einer ersten Stufe photopolymerisiert werden und in einer zweiten Stufe durch thermische Nachbehandlung vernetzt werden.

Die photopolymerisierbaren Gemische können ausser dem Photoinitiator verschiedene Additive enthalten. Beispiele hierfür sind thermische Inhibitoren, die eine vorzeitige Polymerisation verhindern sollen, wie z.B. Hydrochinon oder sterisch gehinderte Phenole. Zur Erhöhung der Dunkellagerstabilität können z.B. Kupferverbindungen, Phosphorverbindungen, quartäre Ammoniumverbindungen oder Hydroxylaminderivate verwendet werden. Zwecks Ausschluss des Luftsauerstoffes während der Polymerisation kann man Paraffin oder ähnliche wachsartige Stoffe zusetzen, die bei Beginn der Polymerisation an die Oberfläche wandern. Als Lichtschutzmittel können in geringer Menge UV-Absorber, wie z.B. solche vom Benztriazol-, Benzophenon- oder Oxalanilid-Typ, zugesetzt werden. Noch besser ist der Zusatz von Lichtschutzmitteln, die UV-Licht nicht absorbieren, wie z.B. von sterisch gehinderten Aminen (HALS).

In bestimmten Fällen kann es von Vorteil sein, Gemische von zwei oder mehr der Photoinitiatoren der Formel IV zu verwenden. Selbstverständlich können auch Gemische mit bekannten Photoinitiatoren verwendet werden, z.B. Gemische mit Benzophenon, Acetophenonderivaten, Benzoinethern, Benzilketalen, Monoacrylphosphinoxiden oder Bisacylphosphinoxiden.

Zur Beschleunigung der Photopolymerisation können Amine zugesetzt werden, wie z.B. Triethanolamin, N-Methyl-diethanolamin, p-Dimethylaminobenzoesäure-ethylester, Michlers Keton oder Bisdiethylaminobenzophenon. Die Wirkung der Amine kann verstärkt werden durch den Zusatz von aromatischen Ketonen vom Typ des Benzophenons.

Eine Beschleunigung der Photopolymerisation kann weiterhin durch Zusatz von Photosensibilisatoren geschehen, welche die spektrale Empfindlichkeit verschieben bzw. verbreitern. Dies sind insbesondere aromatische Carbonylverbindungen wie z.B. Benzophenon-, Thioxanthon-, Anthrachinon- und 3-Acylcumarinderivate sowie 3-(Aroylmethylen)-thiazoline.

Die Wirksamkeit der Photoinitiatoren lässt sich steigern durch Zusatz von Titanocenderivaten mit fluororganischen Resten, wie sie in den EP-A-122'223, 186'626 und 318894 beschrieben sind, z.B. in einer Menge von 0,1 - 20 %. Beispiele für solche Titanocene sind Bis(methylcyclopentadienyl)-bis-(2,3,6-trifluorphenyl)-titan, Bis(cyclopentadienyl)-bis(4-dibutylamino-2,3,5,6-tetrafluorphenyl)-titan, Bis(methylcyclopentadienyl)-2-(trifluormethyl) phenyl-titan-isocyanat, Bis(cyclopentadienyl)-2-(trifluormethyl)phenyl-titan-trifluoracetat, Bis(methylcyclopentadienyl)-bis(4-decyloxy-2,3,5,6-tetrafluorphenyl)-titan, Bis(cyclopentadienyl)-bis-[2,6-difluor-3-(pyrr-1-yl)phenyl]-titan, Bis(methylcyclopentadienyl)-bis-[2,6-difluor-3-(pyrr-1-yl)phenyl]-titan, Bis(cyclopentadienyl)-bis-[2,6-difluor-3-(2,5-dimethyl-pyrr-1-yl)phenyl]-titan und Bis(methylcyclopentadienyl)-bis-[2,6-difluor-3-(2,5-dimethyl-pyrr-1-yl)phenyl]-titan. Für diese Gemische eignen sich vor allem flüssige α-Aminoketone.

Die photopolymerisierbare Zusammensetzung, enthaltend
A) mindestens eine ethylenisch ungesättigte photopolymerisierbare Verbindung, und
B) mindestens einen Photoinitiator der Formel IV sowie
C) gegebenenfalls weitere bekannte und übliche Zusatzstoffe
kann für verschiedene Zwecke verwendet werden. In erster Linie ist ihre Verwendung in pigmentierten oder eingefärbten Systemen von Bedeutung, wie z.B. für Druckfarben, für photographische Reproduktionsverfahren, Bildaufzeichnungsverfahren und zur Herstellung von Reliefformen.

Ein weiteres wichtiges Einsatzgebiet sind Anstrichstoffe, die pigmentiert oder unpigmentiert sein können. Besonders wertvoll sind die Gemische in Weisslacken, worunter man durch TiO₂ pigmentierte Anstrichstoffe versteht. Das in den photohärtbaren Massen enthaltene Pigment kann ein anorganisches Pigment sein, wie z.B. Titandioxid (Rutil oder Anatas), Eisenoxidgelb, Eisenoxidrot, Chromgelb, Chromgrün, Nickeltitangelb, Ultramarinblau, Kobaltblau, Cadmiumgelb, Cadmiumrot oder Zinkweiss. Das Pigment kann ein organisches Pigment sein, wie z.B. ein Mono- oder Bisazopigment oder ein Metallkomplex davon, ein Phthalocyaninpigment, ein polycyclisches Pigment, wie z.B. ein Perylen-, Thioindigo-, Flavanthron-, Chinacridon-, Tetrachlorisoindolinon- oder Triphenylmethan-Pigment. Das Pigment kann auch ein Russ sein oder ein Metallpulver, wie z.B. Aluminium- oder Kupferpulver. Das Pigment kann auch ein Gemisch von zwei oder mehreren verschiedenen Pigmenten sein, wie es zur Erzielung bestimmter Farbtöne üblich ist.

Das Pigment kann in einer Menge von 5 bis 60 Gew.%, bezogen auf die gesamte Masse, vorliegen; in Druckfarben liegen meist 10 - 30 % Pigment vor.

Weitere Einsatzgebiete sind die Strahlenhärtung von Photoresists, die Photovernetzung silberfreier Filme sowie die Herstellung von Druckplatten. Eine weitere Verwendung ist die für Aussenanstriche, die im Tageslicht oberflächlich nachhärten. In Photresist oder reprographischen Filmen werden zur Farbgebung statt Pigmenten auch häufig Farbstoffe verwendet. Hierbei kann es sich um organische Farbstoffe der verschiedensten Klassen handeln, beispielsweise Azofarbstoffe, Methinfarbstoffe, Anthrachinon-Farbstoffe oder Metallkomplexfarbstoffe. Diese Farbstoffe sind in den verwendeten Konzentrationen in den jeweiligen Bindemitteln löslich. Die üblichen Konzentrationen sind 0,1 bis 20 %, vorzugsweise 1-5 Gew.%, bezogen auf die gesamte Masse.

Die Photoinitiatoren werden für die angeführten Anwendungsgebiete zweckmässig in Mengen von 0,1 bis 20 Gew.%, vorzugsweise etwa 0,5 bis 5 Gew.%, bezogen auf die photopolymerisierbare Zusammensetzung, angewendet.

Die Polymerisation erfolgt nach den bekannten Methoden der Photopolymerisation durch Bestrahlung mit Licht, das reich an kurzwelliger Strahlung ist. Als Lichtquellen sind z.B. Quecksilbermitteldruck-, -hochdruck- und -niederdruckstrahler, superaktinische Leuchtstoffröhren, Metallhalogenid-Lampen oder Laser geeignet, deren Emissionsmaxima im Bereich zwischen 250 und 450 nm liegen. Im Falle einer Kombination mit Photosensibilisatoren oder Ferrocenderivaten kann auch längerwelliges Licht oder Laserstrahlen bis 600 nm verwendet werden.

Die folgenden Beispiele veranschaulichen die Erfindung ohne sie darauf zu limitieren.

Beispiel 1:In einem 1 l Hochdruckreaktor werden 181,7 g (0,80 Mol) 1-(4-Bromphenyl)-butan-1-on und 348,5 g (4,0 Mol) Morpholin purum und 72,0 g (4,0 Mol) deionisiertes Wasser vorgelegt. Der Reaktor wird verschlossen und die Lösung wird in ca. 90 Minuten auf 170°C geheizt. Der Innendruck steigt von Null auf 5-6 bar und stabilisiert sich eine Stunde später bei 4-5 bar. Die Reaktionslösung wird ca. 28 Stunden bei ca. 170°C weitergerührt. Dann wird die Reaktionslösung abgekühlt und bei ca. 80°C dem Reaktor entnommen.

Die Reaktionslösung wird in einer Destillationsapparatur auf ca. 104°C erwärmt, um das Wasser abzudestillieren. Dann wird unter schwachem Vakuum das Morpholin abdestilliert. Nach beendeter Destillation wird 144,0 g (0,80 Mol) Natriummethylatlösung à 30 % in Methanol zugesetzt und die Suspension wird aufgeheizt, um das Methanol abzudestillieren. Nach beendeter Methanoldestillation wird die Reaktionsmischung evakuiert und das Morpholin abdestilliert. Bei ca. 80°C werden dann 90 g deionisiertes Wasser zugegeben und verrührt. Das Wasser wird dann abgetrennt. Die überstehende Phase (ca. 196 g Rohausbeute, ca. 105 % der Theorie) wird mit 150 ml (117,5 g) Isopropanol verdünnt, abgekühlt und zur Kristallisation angeimpft. Die Suspension wird bei ca. -10°C filtriert und mit kaltem Isopropanol nachgewaschen. Es werden 148,7 g 1-(4-Morpholinophenyl)-butan-1-on (79,6 % der Theorie) als hellbeige Kristalle mit einem Smp. 64,5°C - 65,5°C erhalten. Reinheit: >99,0 %.

| Elementaranalyse: | | | | | |
|---|---|---|---|---|---|
| | %C | | %H | | % N |
| berechnet | 72,07 | berechnet | 8,21 | berechnet | 6,00 |
| gefunden | 72,03 | gefunden | 8,29 | gefunden | 5,92 |

Aus dem Filtrat (Isopropanol) lassen sich weitere 19,8 g (ca. 10 % der Theorie) an 1-(4-Morpholinophenyl)-butan-1-on gewinnen.

Arbeitet man analog nach der Arbeitsweise von Beispiel 1, verwendet aber äquimolekulare Mengen der cyclischen Amine gemäss Tabelle 1 und äquimolekulare Mengen an 1-(4-Bromphenyl)-alkyl-keton gemäss Tabelle 1 so werden die cyclischen Amin substituierten Phenyl-alkyl-Keton-Verbindungen gemäss der Tabelle 1 erhalten, deren physikalische Analysendaten ebenfalls in der Tabelle 1 angegeben sind.

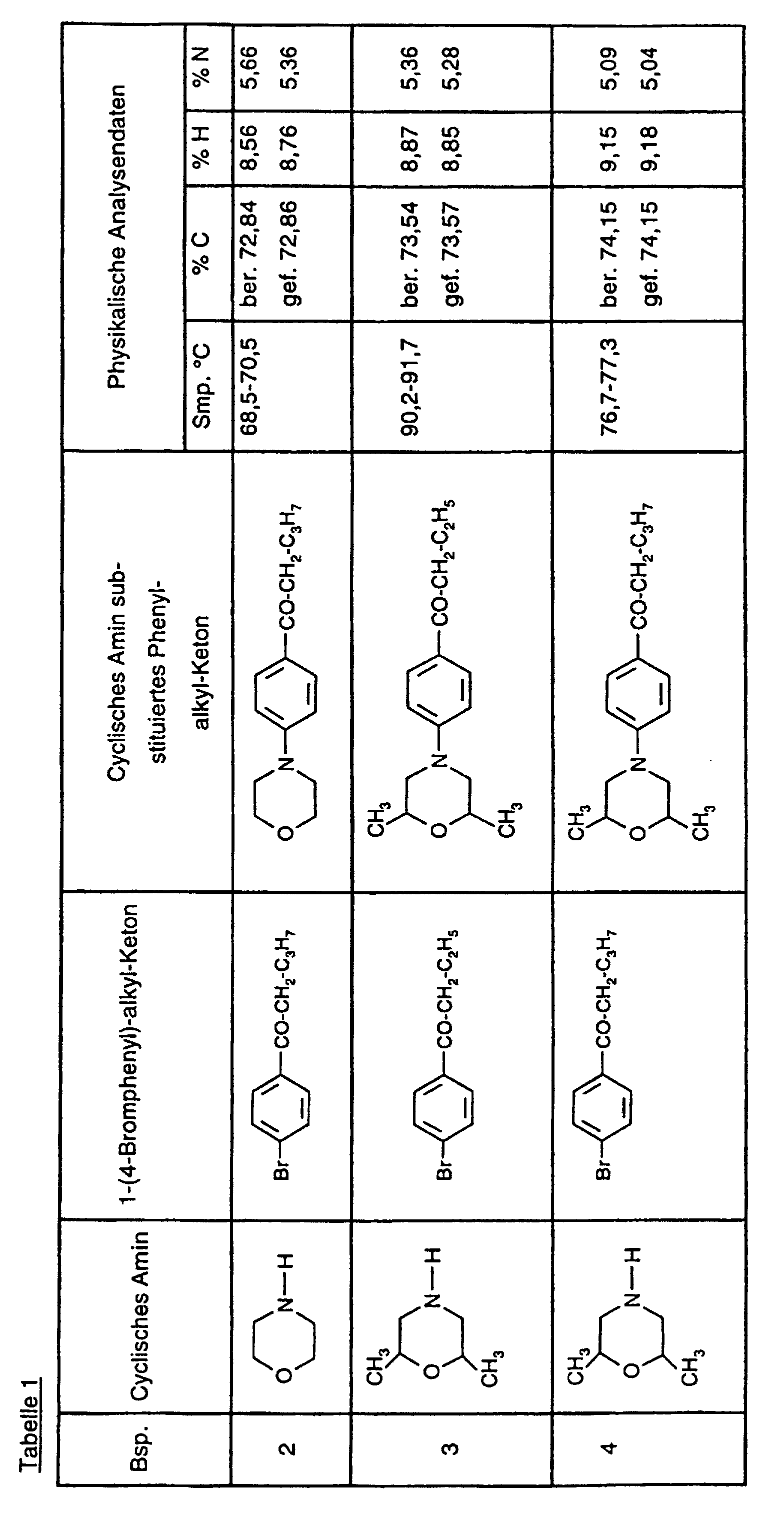

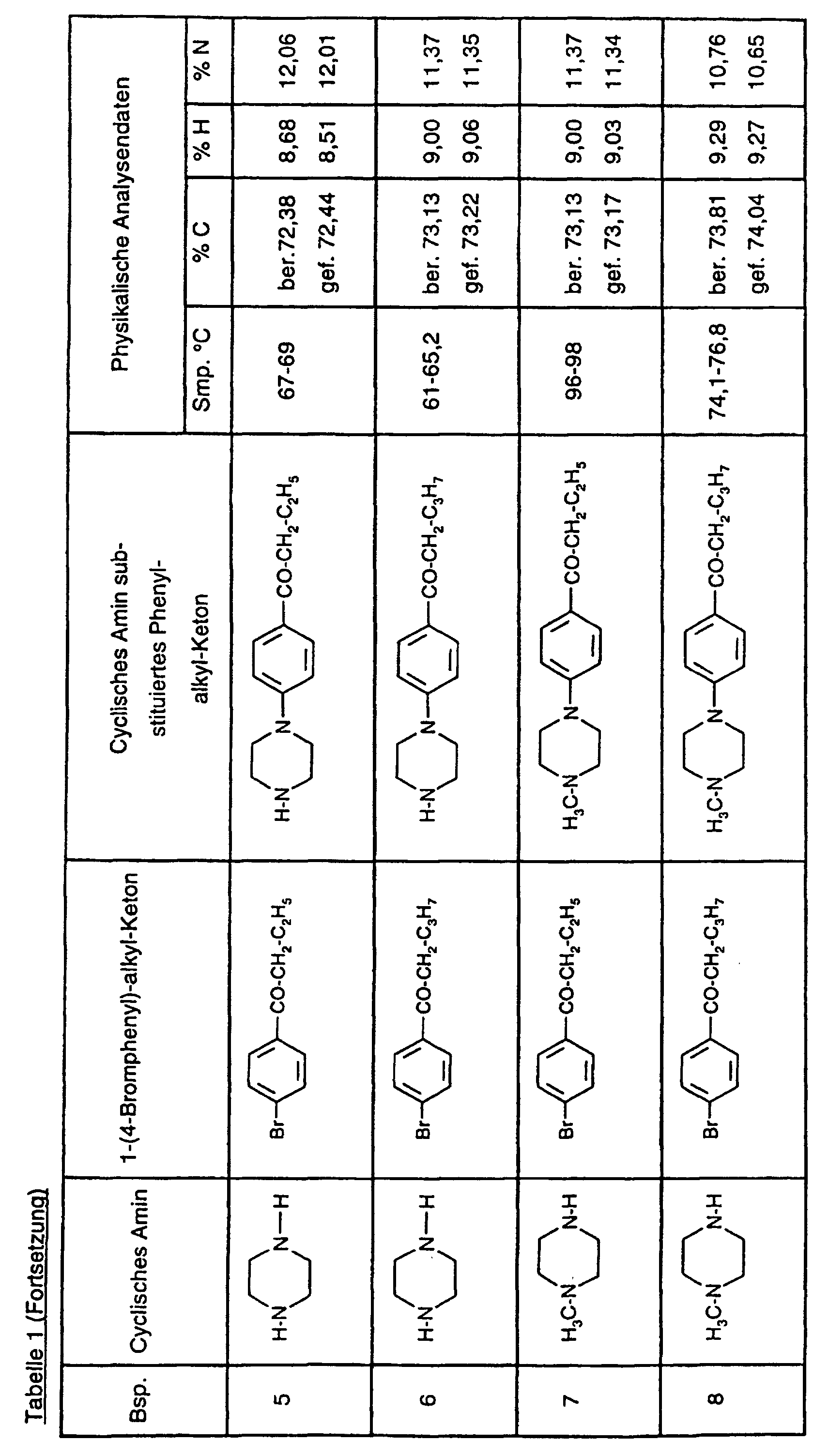

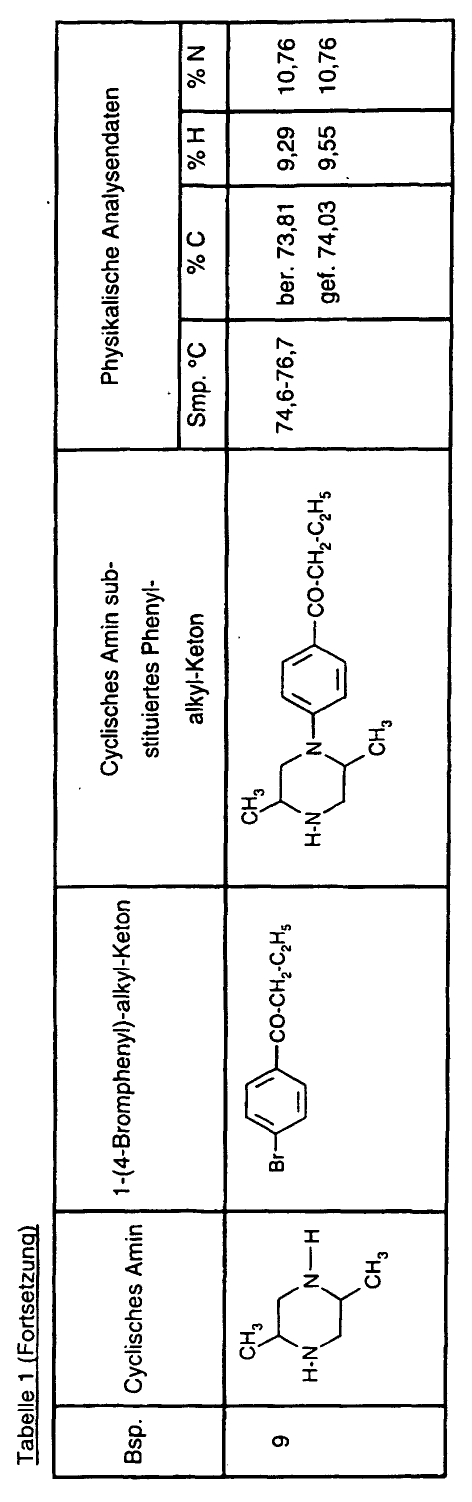

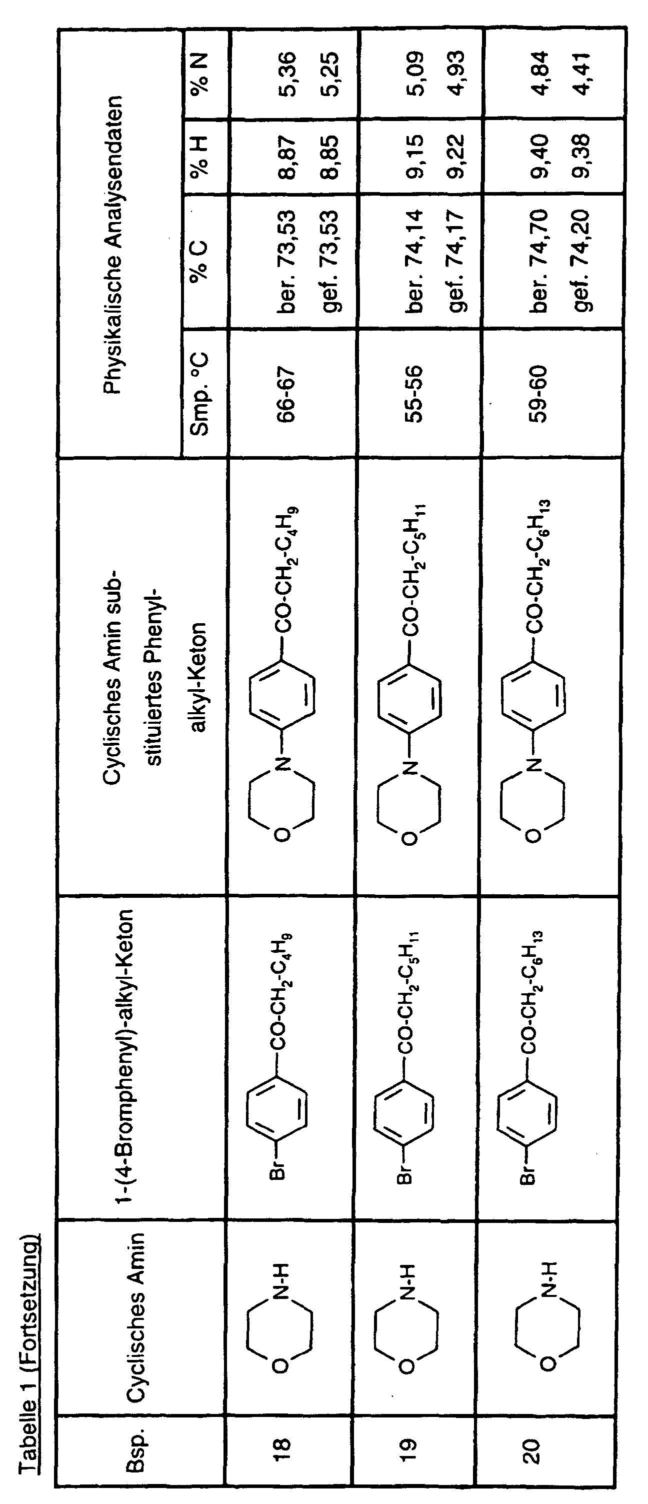

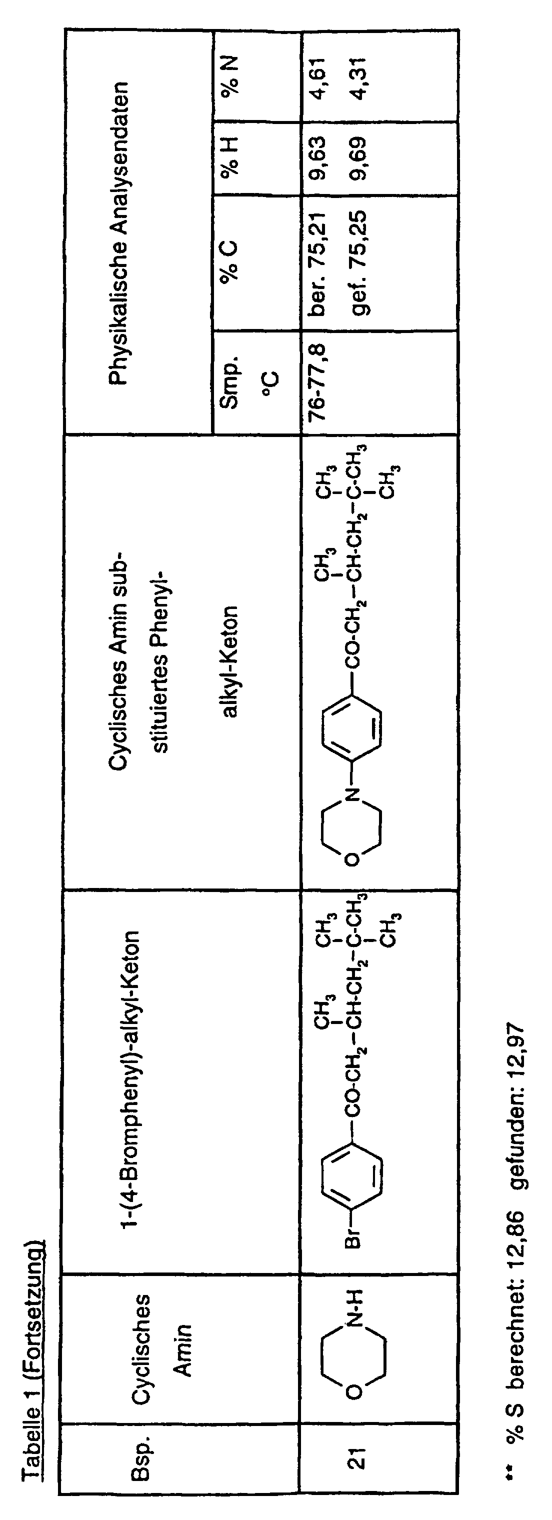

Beispiel 22: In einem 1 l Hochdruckreaktor werden 392,1 g (4,50 Mol) Morpholin purum und 162,0 g (9,00 Mol) deionisiertes Wasser vorgelegt. Der Reaktor wird verschlossen und die Lösung wird unter Rühren in ca. 1 Stunde auf 220°C geheizt, wobei der Innendruck auf 20 bar steigt. Dann werden während fünf Stunden 164,4 g (0,90 Mol) 1-(4-Chlorphenyl) butan-1-on gleichmässig bei 220°C zudosiert. Am Ende des Zudosierens ist der Druck auf ca. 18 bar gefallen und die Reaktion ist über 80 % abgelaufen. Das Reaktionsgemisch wird weitere fünf Stunden bei 220°C nachgerührt. Der Druck sinkt langsam auf 17 bar. Dann lässt man auf 80°C abkühlen.

Mit 75,6 g (0,945 Mol) Natronlauge ä 50 % wird das Morpholinsalz neutralisiert. Unter reduziertem Vakuum wird bei 80°C - 100°C ein Morpholin-Wasser-Gemisch abdestilliert. Dann werden 180 g deionisiertes Wasser und 203 g Siedegrenzenbenzin (110°C - 140°C Siedebereich) zugegeben. Das Gemisch wird bei 80°C über wenig Aktivkohle klarfiltriert. Die Wasserphase wird bei 80°C abgetrennt. Das Produkt wird aus dem Siedegrenzenbenzin auskristallisiert, filtriert und getrocknet. Die Ausbeute an Endprodukt beträgt 200,6 g 1-(4-Morpholinophenyl)-butan-1-on (ca. 95,5 % der Theorie). Das beige Produkt hat eine Reinheit von >99,0 %, der Schmelzpunkt liegt bei 64,8°C. Im Filtrat sind nur Produkt und Edukt feststellbar.

| Elementaranalyse: | | | | | |
|---|---|---|---|---|---|
| | %C | | %H | | % N |
| berechnet | 72,07 | berechnet | 8,21 | berechnet | 6,00 |
| gefunden | 72,09 | gefunden | 8,26 | gefunden | 5,86 |

Beispiel 23:In einem 1 l Hochdruckreaktor werden 392,1 g (4,50 Mol) Morpholin purum und 162,0 g (9,00 Mol) deionisiertes Wasser vorgelegt. Der Reaktor wird verschlossen und in ca. 60 Minuten auf 215°C - 220°C aufgeheizt. Der Druck erreicht 19,9 bar. Dann werden 164,4 g (0,90 Mol) 1-(4-Chlorphenyl)-butan-1-on in flüssiger Form mit einer Druckpumpe zudosiert und die Temperatur bei 215°C - 220°C gehalten.

Dauer der Dosierzeit: 3 Stunden. Der Druck sinkt auf 18,5 bar. Anschliessend wird während 3 weiteren Stunden bei 215°C - 220°C nachgerührt. Der Druck sinkt auf 17,8 bar. Anschliessend wird auf ca. 80°C abgekühlt.

Die Reaktionslösung wird in eine Destillationsapparatur transferiert und mit 36,0 g (0,90 Mol) Natriumhydroxid in Perlenform versetzt. Dann wird das Wasser zwischen 70°C und 90°C unter reduziertem Druck abdestilliert und anschliessend auch das Morpholin. Das Endvacuum ist ca. 30 mbar. Anschliessend wird die Apparatur mit Stickstoff entlastet und es werden bei ca. 88°C 171,8 g deionisiertes Wasser und 30,2 g Toluol zugesetzt. Nach dem Verrühren wird das Wasser abgetrennt und das Toluol abdestilliert. Die Reaktionslösung wird warm mit 152,9 g Isopropanol versetzt und bei ca. 65°C heiss über einen Druckfilter geklärt. Die Isopropanollösung wird gekühlt und angeimpft. Die Suspension wird bei ca. 0°C filtriert und mit kaltem Isopropanol nachgewaschen. Es werden 186,7 g 1-(4-Morpholinophenyl)-butan-1-on (88,9 % der Theorie) als hellbeige Kristalle mit einem Smp.: 64,4°C - 65,5°C erhalten.

Beispiel 24:In einem 1 l Hochdruckreaktor werden 164,4 g (0,90 Mol) 1-(4-Chlorphenyl)-butan-1-on, 392,1 g (4,50 Mol) Morpholin purum, 162,0 g (9,00 Mol) deionisiertes Wasser und 0,89 g (0,90 mMol) Kupfer-1-chlorid vorgelegt. Der Reaktor wird verschlossen und die Lösung wird unter Rühren in ca. 1 Stunde auf 180°C geheizt. Dann wird langsam weitergeheizt und pro Stunde die Temperatur um ca. 10°C erhöht. In vier Stunden werden 220°C und ein Druck von 20 bar erreicht. Dann lässt man weitere fünf Stunden bei 220°C nachreagieren. Der Druck sinkt dabei langsam auf 17 bar. Dann lässt man auf 80°C abkühlen.

Mit 75,6 g (0,945 Mol) Natronlauge à 50 % wird das Morpholinsalz neutralisiert und der Katalysator gefällt. Unter reduziertem Vakuum wird bei 80°C - 100°C ein Morpholin-Wasser-Gemisch abdestilliert. Dann werden 180 g deionisiertes Wasser und 203 g Siedegrenzenbenzin (110°C - 140°C Siedebereich) zugegeben. Das Gemisch wird bei 80°C über wenig Aktivkohle klarfiltriert um den Katalysator zu entfernen. Die Wasserphase wird bei 80°C abgetrennt. Das Produkt wird aus dem Siedegrenzenbenzin auskristallisiert, filtriert und getrocknet. Die Ausbeute an Endprodukt beträgt 199,8 g 1-(4-Morpholinophenyl)-butan-1-on (ca. 95,2 % der Theorie). Das beige Produkt besitzt eine Reinheit von >99,0 %, der Schmelzpunkt liegt bei 64,8°C. Im Filtrat sind nur Produkt und Edukt feststellbar.

### Beispiel 25:

### a) 2-Brom-1-(4-morpholinophenyl)-butan-1-on

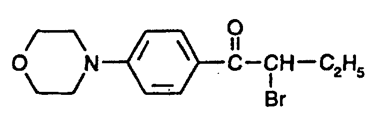

In einem 2,5 l Sulfierkolben werden 466,6 g (2 Mol) 1-(4-Morpholinophenyl)-butan-1-on gemäss Beispiel 1 in 600 ml (10,5 Mol) Eisessig gelöst. Die Temperatur sinkt dabei auf 5°C. Unter schwacher Kühlung werden in ca. 2,5 Stunden bei Raumtemperatur 319,6 g (2 Mol) Brom zugetropft. Das Ende der Bromierung wird mit einem Dünnschichtchromatogramm überprüft. Dann wird die Reaktionslösung mit 300 g Eis versetzt und dazu wird unter guter Kühlung in einer Stunde eine Natronlaugenlösung, hergestellt aus 1600 g (12 Mol) Natronlauge und 600 g Eis, zugetropft. Die gelbe Suspension hat ca. pH 6, wird filtriert und mit Wasser nachgewaschen. Die Kristalle werden getrocknet. Sie schmelzen bei 99°C bis 102°C. Die Ausbeute ist 631,2 g von 2-Brom-1-(4-morpholinophenyl)-butan-1-on. Das ¹H-NMR-Spektrum des Rohproduktes stimmt mit der angegebenen Struktur überein.

| Elementaranalyse | %C | %H | %N | %Br |
|---|---|---|---|---|
| berechnet | 53,86 | 5,81 | 4,49 | 25,59 |
| gefunden | 53,23 | 5,73 | 4,24 | 25,50 |

### b) 2-Dimethylamino-1-(4-morpholinophenyl)-butan-1-on

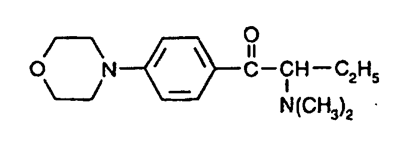

In einem 2,5 l Sulfierkolben werden 312,2 g (1 Mol) 2-Brom-1-(4-morpholinophenyl) butan-1-on gemäss a) oben mit 600 ml Methylethylketon versetzt und unter Rühren auf 50°C erwärmt. Zur erhaltenen Lösung gibt man 207,3 g (1,5 Mol) Kaliumcarbonat und leitet bei 50°C innerhalb 1,5 Stunden unter Niveau 56,6 g (1,3 Mol) gasförmiges Dimethylamin in die Suspension ein. Dann lässt man noch weitere 4 bis 5 Stunden nachreagieren, bis im Dünnschichtchromatogramm kein Edukt mehr nachweisbar ist. Die Suspension wird dann mit 550 ml Wasser versetzt und verrührt. Die wässrige Phase wird abgetrennt und die 900 ml organische Phase, enthaltend 2-Dimethylamino-1-(4-morpholinophenyl)-butan-1-on, wird unverändert in der nächsten Reaktionsstufe eingesetzt.

In einem Parallelversuch wird die organische Phase eingeengt. Die erhaltenen Kristalle werden aus Hexan umkristallisiert. Nach dem Trocknen erhält man 235,1 g hellgelbe Kristalle, die bei 53°C bis 56°C schmelzen. Das ¹H-NMR-Spektrum des Produktes, 2-Dimethylamino-1-(4-morpholinophenyl)-butan-1-on, stimmt mit der angegebenen Struktur überein.

| Elementaranalyse | %C | %H | %N |
|---|---|---|---|
| berechnet | 69,53 | 8,75 | 10,14 |
| gefunden | 68,91 | 8,59 | 9,74 |

### c) 2-Benzyl-2-dimethylamino-1-(4-morpholinophenyl)-butan-1-on

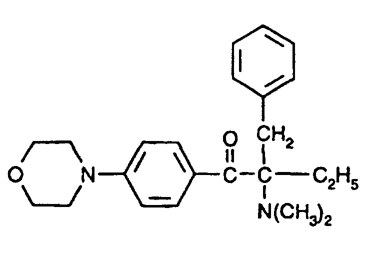

In einem 2,5 l Sulfierkolben werden 900 ml Lösung (1 Mol) von 2-Dimethylamino-1-(4-morpholinophenyl)-butan-1-on gemäss b) oben wieder auf 50°C erwärmt. Dann werden in 20 Minuten 179,7 g (1,05 Mol) Benzylbromid zugetropft. Dann lässt man noch weitere 3 bis 4 Stunden bei 50°C nachrühren, bis im Dünnschichtchromatogramm kein Edukt mehr nachweisbar ist. Nun wird die Temperatur auf 60°C erhöht und innert 45 Minuten werden portionenweise 80 g (2 Mol) Natriumhydroxidpulver zugegeben. Anschliessend wird noch 1 bis 2 Stunden bei 50°C weitergerührt, bis im Dünnschichtchromatogramm kein Edukt mehr nachweisbar ist. Das Reaktionsgemisch wird mit 150 ml Wasser versetzt und verrührt. Die Wasserphase wird abgetrennt und die organische Phase am Vacuumrotationsverdampfer eingeengt. Im Kolben bleiben 378,3 g gelbliches Rohprodukt von 2-Benzyl-2-dimethylamino-1-(4-morpholinophenyl)-butan-1-on, das bei 102°C bis 110°C schmilzt. Das Rohprodukt wird in 600 ml Ethanol heiss gelöst, abgekühlt, kristallisiert, filtriert und mit kaltem Ethanol nachgewaschen. Die Kristalle werden getrocknet. Sie schmelzen bei 114°C bis 115°C und sind im Gas- und im Dünnschicht-chromatogramm rein. Die Ausbeute ist 299,0 g von 2-Benzyl-2-dimethylamino-1-(4-morpholinophenyl)-butan-1-on. Aus der Mutterlauge lassen sich weitere 22,4 g an reinem Produkt isolieren. Das ¹H-NMR-Spektrum des Reinproduktes stimmt mit der angegebenen Struktur überein.

| Elementaranalyse | %C | %H | %N |
|---|---|---|---|
| berechnet | 75,38 | 8,25 | 7,64 |
| gefunden | 75,23 | 8,21 | 7,58 |

## Patentansprüche

1. Die Verbindungen
1-(4-morpholinophenyl)butan-1-one, 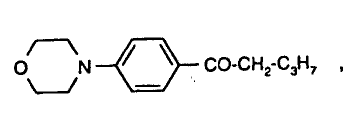 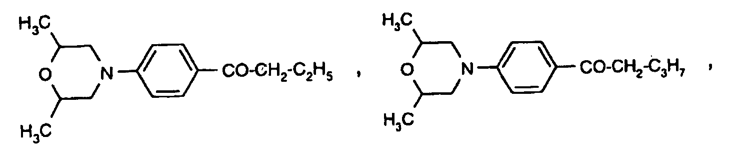 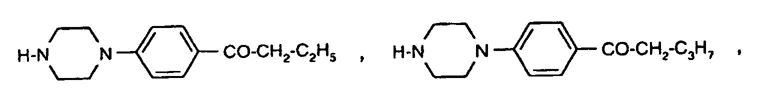 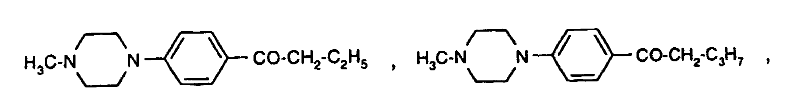 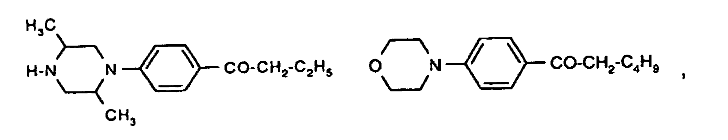 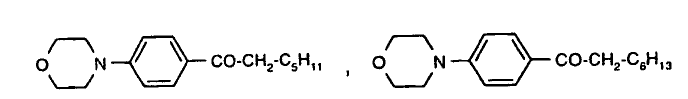 und

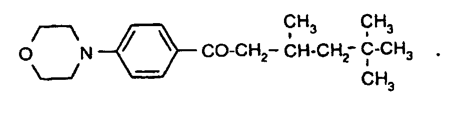

2. Verwendung der Verbindungen der Formel I 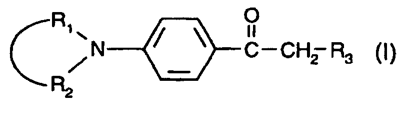 worin bedeuten:
R₁ und R₂ zusammen unter Einschluss des N-Atoms einen Morpholinyl, Dimethylmorpholinyl-, Piperazinyl-, N-Methylpiperazinyl- oder 2,5-Dimethylpiperazinylrest;
R₃ unverzweigtes oder verzweigtes, unsubstituiertes oder substituiertes C₂-C₂₀-Alkyl;
zur Herstellung von Photoinitiatoren der Formel IV 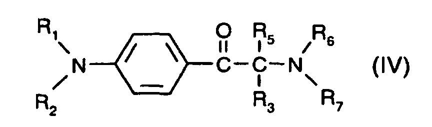 oder deren Säureadditionssalze, worin bedeuten:
R₁ und R₂ zusammen unter Einschluss des N-Atoms einen Morpholinyl, Dimethylmorpholinyl-, Piperazinyl-, N-Methylpiperazinyl- oder 2,5-Dimethylpiperazinylrest;
R₃ unverzweigtes oder verzweigtes, unsubstituiertes oder durch C₁-C₄-Alkoxy, Phenoxy, Cyclohexyl oder Phenyl substituiertes C₂-C₂₀-Alkyl;
R₅ entweder
(a) ein Rest der Formel 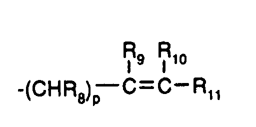 worin p null oder 1 ist,
oder
(b) ein Rest der Formel 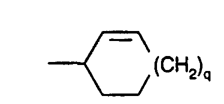 ist, wobei q 0, 1, 2 oder 3 bedeutet
oder
c) ein Rest der Formel 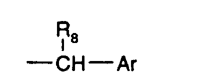
worin Ar einen unsubstituierten oder durch Halogen, OH, C₁-C₁₂-Alkyl, oder durch OH, Halogen, -N(R₁₂)₂, -C₁-C₁₂-Alkoxy, -COO(C₁-C₁₈-Alkyl), -CO(OCH₂CH₂)ₙOCH₃ oder -OCO(C₁-C₄)-Alkyl substituiertes C₁-C₄-Alkyl; C₁-C₁₂-Alkoxy, oder durch -COO(C₁-C₁₈-Alkyl) oder -CO(OCH₂CH₂)ₙOCH₃ substituiertes C₁-C₄-Alkoxy; -(OCH₂CH₂)ₙOH, -(OCH₂CH₂)ₙOCH₃, C₁-C₈-Alkylthio, Phenoxy, -COO(C₁-C₁₈-Alkyl), -CO(OCH₂CH₂)ₙOCH₃, Phenyl oder Benzoyl substituierten Phenyl-, Naphthyl-, Furyl-, Thienyl- oder Pyridylrest bedeutet, worin n 1 - 20 ist,
in welchen Formeln
R₁₂ Wasserstoff, C₁-C₈-Alkyl, C₃-C₅-Alkenyl, C₇-C₉-Phenylalkyl, C₁-C₄-Hydroxyalkyl oder Phenyl bedeutet,
R₈ Wasserstoff, C₁-C₈-Alkyl oder Phenyl bedeutet, und
R₉, R₁₀ und R₁₁ unabhängig voneinander Wasserstoff oder C₁-C₄-Alkyl bedeuten oder R₉ und R₁₀ zusammen C₃-C₇-Alkylen sind,
R₆ Wasserstoff, C₁-C₁₂-Alkyl, durch Hydroxy, C₁-C₄-Alkoxy, -CN oder -COO(C₁-C₄-Alkyl) substituiertes C₂-C₄-Alkyl; C₃-C₅-Alkenyl, C₅-C₁₂-Cycloalkyl oder C₇-C₉-Phenylalkyl bedeutet, R₇ C₁-C₁₂-Alkyl, durch Hydroxy, C₁-C₄-Alkoxy, -CN oder -COO(C₁-C₄-Alkyl) substituiertes C₂-C₄-Alkyl; C₃-C₅-Alkenyl, C₅-C₁₂-Cycloalkyl, C₇-C₉-Phenylalkyl, Phenyl oder durch Halogen, C₁-C₁₂-Alkyl, C₁-C₄-Alkoxy oder -COO(C₁-C₄-Alkyl) substituiertes Phenyl bedeutet oder R₇ zusammen mit R₃ C₁-C₇-Alkylen, C₇-C₁₀-Phenylalkylen, o-Xylylen, 2-Butenylen oder C₂-C₃-Oxa- oder Azaalkylen bedeutet, oder
R₆ und R₇ zusammen C₃-C₇-Alkylen bedeuten, das durch -O-, -S-, -CO- oder -N(R₁₃)-unterbrochen sein kann oder durch Hydroxy, C₁-C₄-Alkoxy oder -COO(C₁-C₄-Alkyl) substituiert sein kann, wobei R₁₃ Wasserstoff, C₁-C₁₂-Alkyl, das durch ein oder mehrere -O-unterbrochen sein kann, C₃-C₅-Alkenyl, C₇-C₉-Phenylalkyl, C₁-C₄-Hydroxyalkyl, -CH₂CH₂CN, -CH₂CH₂COO(C₁-C₄-Alkyl), C₂-C₈-Alkanoyl oder Benzoyl bedeutet.

3. Verfahren zur Herstellung von Photoinitiatoren der Formel IV 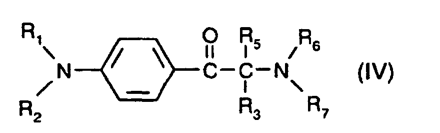 oder deren Säureadditionssalze, worin bedeuten:
R₁ und R₂ zusammen unter Einschluss des N-Atoms einen Morpholinyl, Dimethylmorpholinyl-, Piperazinyl-, N-Methylpiperazinyl- oder 2,5-Dimethylpiperazinylrest;
R₃ unverzweigtes oder verzweigtes, unsubstituiertes oder durch C₁-C₄-Alkoxy, Phenoxy, Cyclohexyl oder Phenyl substituiertes C₂-C₂₀-Alkyl;
R₅ entweder
(a) ein Rest der Formel 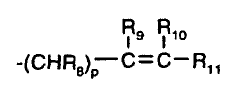 worin p null oder 1 ist,
oder
(b) ein Rest der Formel 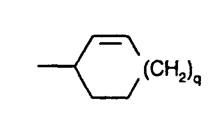 ist, wobei q 0, 1, 2 oder 3 bedeutet
oder
c) ein Rest der Formel 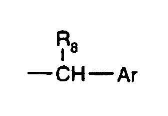
worin Ar einen unsubstituierten oder durch Halogen, OH, C₁-C₁₂-Alkyl, oder durch OH, Halogen, -N(R₁₂)₂, -C₁-C₁₂-Alkoxy, -COO(C₁-C₁₈-Alkyl), -CO(OCH₂CH₂)ₙOCH₃ oder -OCO(C₁-C₄)-Alkyl substituiertes C₁-C₄-Alkyl; C₁-C₁₂-Alkoxy, oder durch -COO(C₁-C₁₈-Alkyl) oder -CO(OCH₂CH₂)ₙOCH₃ substituiertes C₁-C₄-Alkoxy; -(OCH₂CH₂)ₙOH, -(OCH₂CH₂)ₙOCH₃, C₁-C₈-Alkylthio, Phenoxy, -COO(C₁-C₁₈-Alkyl), -CO(OCH₂CH₂)ₙOCH₃, Phenyl oder Benzoyl substituierten Phenyl-, Naphthyl-, Furyl-, Thienyl- oder Pyridylrest bedeutet, worin n 1 - 20 ist,
in welchen Formeln
R₁₂ Wasserstoff, C₁-C₈-Alkyl, C₃-C₅-Alkenyl, C₇-C₉-Phenylalkyl, C₁-C₄-Hydroxyalkyl oder Phenyl bedeutet,
R₈ Wasserstoff, C₁-C₈-Alkyl oder Phenyl bedeutet, und
R₉, R₁₀ und R₁₁ unabhängig voneinander Wasserstoff oder C₁-C₄-Alkyl bedeuten oder R₉ und R₁₀ zusammen C₃-C₇-Alkylen sind,
R₆ Wasserstoff, C₁-C₁₂-Alkyl, durch Hydroxy, C₁-C₄-Alkoxy, -CN oder -COO(C₁-C₄-Alkyl) substituiertes C₂-C₄-Alkyl; C₃-C₅-Alkenyl, C₅-C₁₂-Cycloalkyl oder C₇-C₉-Phenylalkyl bedeutet, R₇ C₁-C₁₂-Alkyl, durch Hydroxy, C₁-C₄-Alkoxy, -CN oder -COO(C₁-C₄-Alkyl) substituiertes C₂-C₄-Alkyl; C₃-C₅-Alkenyl, C₅-C₁₂-Cycloalkyl, C₇-C₉-Phenylalkyl, Phenyl oder durch Halogen, C₁-C₁₂-Alkyl, C₁-C₄-Alkoxy oder -COO(C₁-C₄-Alkyl) substituiertes Phenyl bedeutet oder R₇ zusammen mit R₃ C₁-C₇-Alkylen, C₇-C₁₀-Phenylalkylen, o-Xylylen, 2-Butenylen oder C₂-C₃-Oxa- oder Azaalkylen bedeutet, oder
R₆ und R₇ zusammen C₃-C₇-Alkylen bedeuten, das durch -O-, -S-, -CO- oder -N(R₁₃)-unterbrochen sein kann oder durch Hydroxy, C₁-C₄-Alkoxy oder -COO(C₁-C₄-Alkyl) substituiert sein kann, wobei R₁₃ Wasserstoff, C₁-C₁₂-Alkyl, das durch ein oder mehrere -O-unterbrochen sein kann, C₃-C₅-Alkenyl, C₇-C₉-Phenylalkyl, C₁-C₄-Hydroxyalkyl, -CH₂CH₂CN, -CH₂CH₂COO(C₁-C₄-Alkyl), C₂-C₈-Alkanoyl oder Benzoyl bedeutet,
durch Aminolyse eines p-Halogenphenylalkylketons der Formel II 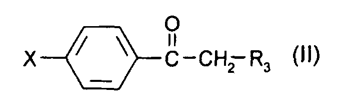 mit einem cyclischen Amin der Formel III 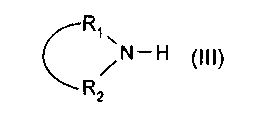 in Wasser bei einer Temperatur, von mindestens 130°C in welchen Formeln X ein Halogenatom bedeutet, und R₁, R₂ und R₃ die oben angegebene Bedeutung haben, zu einem cyclischen Amin substituierten Phenyl-alkyl-Keton der Formel 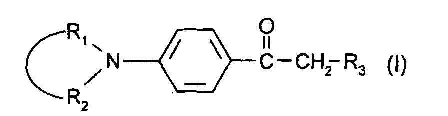 Halogenierung dieser Phenyl-alkyl-Keton Verbindung der Formel I, Umsetzung mit einem Amin der Formel 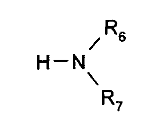 anschliessender Umsetzung mit einer den Rest R₅ einführenden Verbindung und Stevens-Umlagerung unter basischen Konditionen.

## Claims

1. A compound
1-(4-morpholinophenyl)butan-1-one, 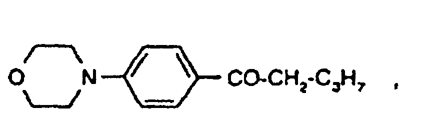 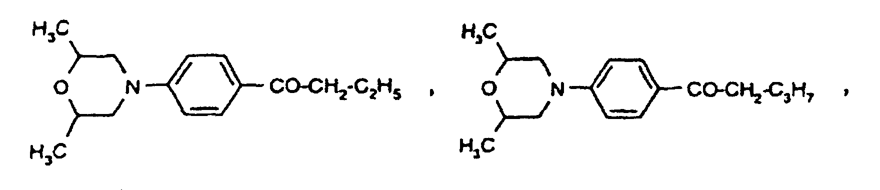 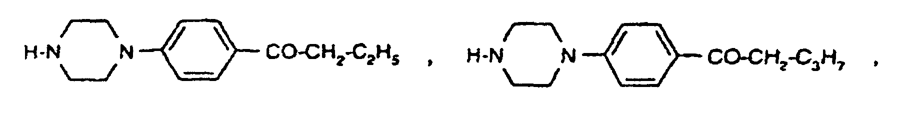 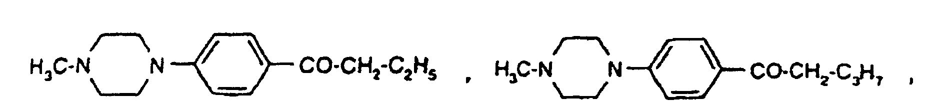 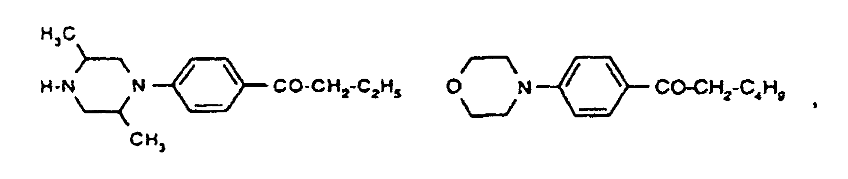 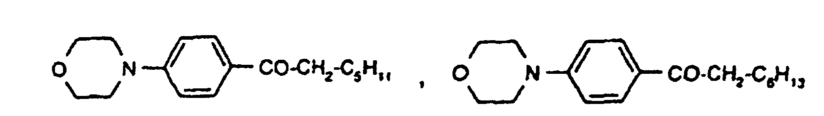 or

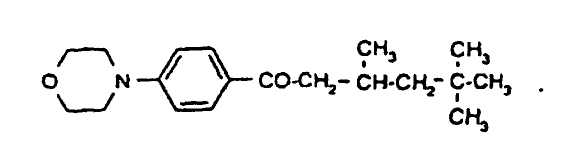

2. Use of a compound of formula I 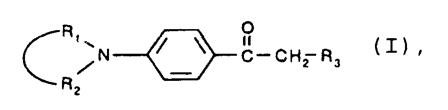 in which:
R₁ and R₂ together, with inclusion of the N atom, are a morpholinyl, dimethylmorpholinyl, piperazinyl, N-methylpiperazinyl or 2,5-dimethylpiperazinyl radical;
R₃ is straight-chain or branched, unsubstituted or substituted C₂-C₂₀alkyl; for preparing a photoinitiator of formula IV 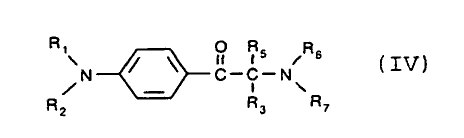
or an acid addition salt thereof, in which:
R₁ and R₂ together, with inclusion of the N atom, are a morpholinyl, dimethylmorpholinyl, piperazinyl, N-methylpiperazinyl or 2,5-dimethylpiperazinyl radical;
R₃ is straight-chain or branched C₂-C₂₀alkyl which is unsubstituted or substituted by C₁-C₄alkoxy, phenoxy, cyclohexyl or phenyl;
R₅ is either
(a) a radical of formula 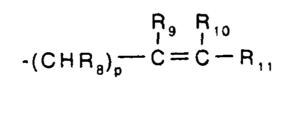 in which p is zero or 1,
or
(b) a radical of formula 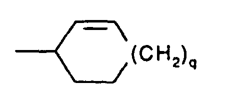 in which q is 0, 1, 2 or 3,
or
(c) a radical of formula 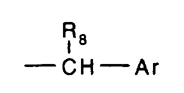
in which Ar is a phenyl, naphthyl, furyl, thienyl or pyridyl radical which is unsubstituted or substituted by halogen, OH, C₁-C₁₂alkyl, or by C₁-C₄alkyl which is substituted by OH, halogen, -N(R₁₂)₂, -C₁-C₁₂alkoxy, -COO(C₁-C₁₈alkyl), -CO(OCH₂CH₂)ₙOCH₃ or -OCO(C₁-C₄)alkyl; by C₁-C₁₂alkoxy, or by C₁-C₄alkoxy which is substituted by -COO(C₁-C₁₈alkyl) or -CO(OCH₂CH₂)ₙOCH₃; or by -(OCH₂CH₂)ₙOH, -(OCH₂CH₂)ₙOCH₃, C₁-C₈alkylthio, phenoxy, -COO(C₁-C₁₈alkyl), -CO(OCH₂CH₂)ₙOCH₃, phenyl or benzoyl, in which n is 1 - 20,
in which formulae
R₁₂ is hydrogen, C₁-C₈alkyl, C₃-C₅alkenyl, C₇-C₉phenylalkyl, C₁-C₄hydroxyalkyl or phenyl,
R₈ is hydrogen, C₁-C₈alkyl or phenyl, and
R₉, R₁₀ and R₁₁ are each independently of one another hydrogen or C₁-C₄alkyl, or R₉ and R₁₀ together are C₃-C₇alkylene,
R₆ is hydrogen, C₁-C₁₂alkyl; C₂-C₄alkyl which is substituted by hydroxyl, C₁-C₄alkoxy, -CN or -COO(C₁-C₄alkyl);
C₃-C₅alkenyl, C₅-C₁₂cycloalkyl or C₇-C₉phenylalkyl,
R₇ is C₁-C₁₂alkyl; C₂-C₄alkyl which is substituted by hydroxyl, C₁-C₄alkoxy, -CN or -COO(C₁-C₄alkyl); C₃-C₅alkenyl, C₅-C₁₂cycloalkyl, C₇-C₉phenylalkyl, phenyl, or phenyl which is substitued by halogen, C₁-C₁₂alkyl, C₁-C₄alkoxy or -COO(C₁-C₄alkyl) , or R₇ together with R₃ is C₁-C₇alkylene, C₇-C₁₀-phenylalkylene, o-xylylene, 2-butenylene or C₂-C₃oxaalkylene or azaalkylene, or
R₆ and R₇ together are C₃-C₇alkylene which may be interrupted by -O-, -S-, -CO- or -N(R₁₃)- or which may be substituted by hydroxyl, C₁-C₄alkoxy or -COO(C₁-C₄alkyl), where R₁₃ is hydrogen, C₁-C₁₂alkyl which may be interrupted by one or more than one -O-; C₃-C₅alkenyl, C₇-C₉phenylalkyl, C₁-C₄hydroxyalkyl, -CH₂CH₂CN, -CH₂CH₂COO(C₁-C₄alkyl), C₂-C₈alkanoyl or benzoyl.

3. A process for the preparation of a photoinitiator of formula IV 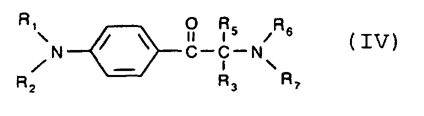 or an acid addition salt thereof, in which:
R₁ and R₂ together, with inclusion of the N atom, are a morpholinyl, dimethylmorpholinyl, piperazinyl, N-methylpiperazinyl or 2,5-dimethylpiperazinyl radical;
R₃ is straight-chain or branched C₂-C₂₀alkyl which is unsubstituted or substituted by C₁-C₄alkoxy, phenoxy, cyclohexyl or phenyl;
R₅ is either
(a) a radical of formula 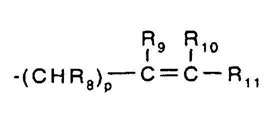 in which p is zero or 1,
or
(b) a radical of formula 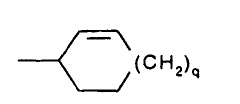 in which q is 0, 1, 2 or 3,
or
(c) a radical of formula 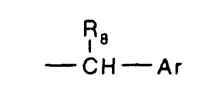
in which Ar is a phenyl, naphthyl, furyl, thienyl or pyridyl radical which is unsubstituted or substituted by halogen, OH, C₁-C₁₂alkyl, or by C₁-C₄alkyl which is substituted by OH, halogen, -N(R₁₂)₂, -C₁-C₁₂alkoxy, -COO(C₁-C₁₈alkyl), -CO(OCH₂CH₂)ₙOCH₃ or -OCO(C₁-C₄)alkyl; by C₁-C₁₂alkoxy, or by C₁-C₄alkoxy which is substituted by -COO(C₁-C₁₈alkyl) or -CO(OCH₂CH₂)ₙOCH₃; or by - (OCH₂CH₂)ₙOH, -(OCH₂CH₂)ₙOCH₃, C₁-C₈alkylthio, phenoxy, -COO(C₁-C₁₈alkyl), -CO(OCH₂CH₂)ₙOCH₃, phenyl or benzoyl, in which n is 1 - 20,
in which formulae
R₁₂ is hydrogen, C₁-C₈alkyl, C₃-C₅alkenyl, C₇-C₉phenylalkyl, C₁-C₄hydroxyalkyl or phenyl,
R₈ is hydrogen, C₁-C₈alkyl or phenyl, and
R₉, R₁₀ and R₁₁ are each independently of one another hydrogen or C₁-C₄alkyl, or R₉ and R₁₀ together are C₃-C₇alkylene,
R₆ is hydrogen, C₁-C₁₂alkyl; C₂-C₄alkyl which is substituted by hydroxyl, C₁-C₄alkoxy, -CN or -COO(C₁-C₄alkyl); C₃-C₅alkenyl, C₅-C₁₂cycloalkyl or C₇-C₉phenylalkyl,
R₇ is C₁-C₁₂alkyl; C₂-C₄alkyl which is substituted by hydroxyl, C₁-C₄alkoxy, -CN or -COO(C₁-C₄alkyl); C₃-C₅alkenyl, C₅-C₁₂cycloalkyl, C₇-C₉phenylalkyl, phenyl, or phenyl which is substitued by halogen, C₁-C₁₂alkyl, C₁-C₄alkoxy or -COO(C₁-C₄alkyl), or R₇ together with R₃ is C₁-C₇alkylene, C₇-C₁₀-phenylalkylene, o-xylylene, 2-butenylene or C₂-C₃oxaalkylene or azaalkylene, or
R₆ and R₇ together are C₃-C₇alkylene which may be interrupted by -O-, -S-, -CO- or -N(R₁₃)- or which may be substituted by hydroxyl, C₁-C₄alkoxy or -COO(C₁-C₄alkyl), where R₁₃ is hydrogen, C₁-C₁₂alkyl which may be interrupted by one or more than one -O-; C₃-C₅alkenyl, C₇-C₉phenylalkyl, C₁-C₄hydroxyalkyl, -CH₂CH₂CN, -CH₂CH₂COO(C₁-C₄alkyl), C₂-C₈alkanoyl or benzoyl
by aminolysis of a p-halophenyl alkyl ketone of formula II 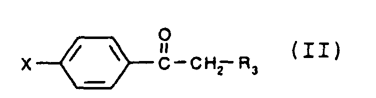 with a cyclic amine of formula III 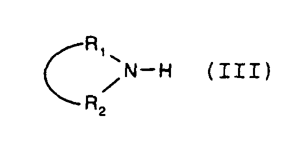 in water at a temperature of at least 130°C, in which formulae X is a halogen atom and R₁, R₂ and R₃ are as defined above, to give a cyclic amine-substituted phenyl alkyl ketone of formula I 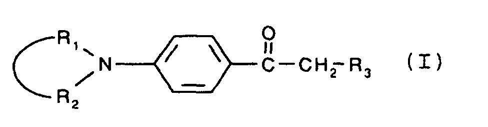 halogenation of this phenyl alkyl ketone compound of formula I, reaction with an amine of formula 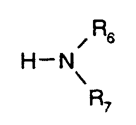 subsequent reaction with a compound introducing the radical R₅, and Stevens rearrangement under basic conditions.

## Revendications

1. Composés
1-(4-morpholinophényl)butan-1-one, 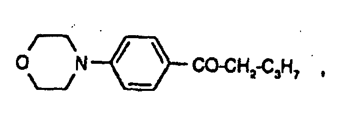 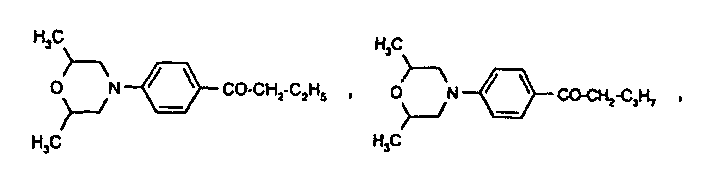 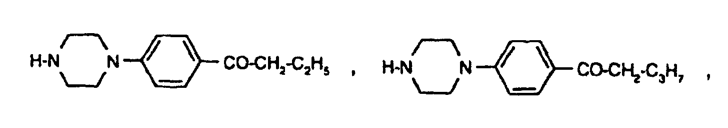 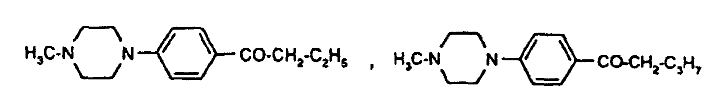 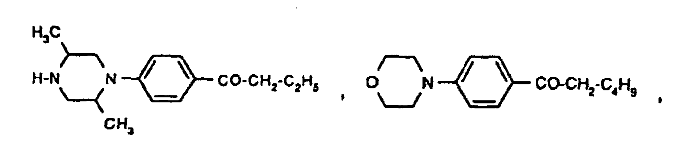 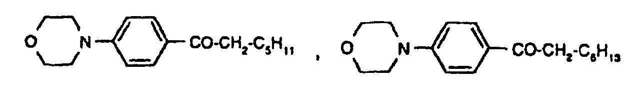 et

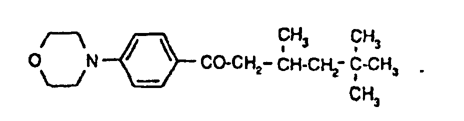

2. Utilisation de composés de formule I 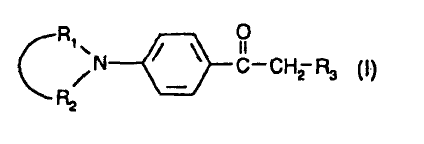 dans laquelle :
R₁ et R₂ représentent conjointement, atome d'azote compris, un groupe morpholinyle, diméthylmorpholinyle, pipérazinyle, N-méthylpipérazinyle ou 2,5-diméthylpipérazinyle ; et
R₃ représente un groupe alkyle en C₂ à C₂₀ ramifié ou non ramifié, substitué ou non substitué ;
pour la production de photoamorceurs de formule IV 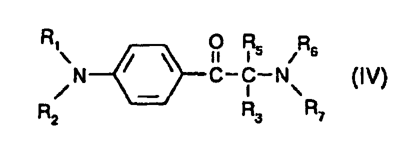 ou de leurs sels d'addition d'acide, dans laquelle :
R₁ et R₂ représentent conjointement, atome d'azote compris, un groupe morpholinyle, diméthylmorpholinyle, pipérazinyle, N-méthylpipérazinyle ou 2,5-diméthylpipérazinyle ;
R₃ représente un groupe alkyle en C₂ à C₂₀ ramifié ou non ramifié, non substitué ou substitué par un groupe alcoxy en C₁ à C₄, phénoxy, cyclohexyle ou phényle ;
R₅ représente
(a) un groupe de formule 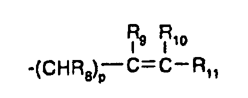 dans laquelle p est égal à zéro ou 1,
ou
(b) représente un groupe de formule 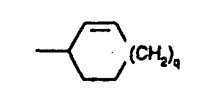 où q représente 0, 1, 2 ou 3, ou
c) représente un groupe de formule 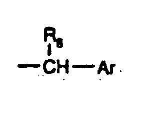
dans laquelle Ar représente un groupe alkyle en C₁ à C₁₂ non substitué ou substitué par un atome d'halogène, un groupe OH, ou un groupe alkyle en C₁ à C₄ substitué par un groupe OH, un atome d'halogène, -N(R₁₂)₂, un groupe alcoxy en C₁ à C₁₂, -COO(alkyle en C₁ à C₁₈), -CO(OCH₂CH₂)ₙOCH₃ ou -OCO(alkyle en C₁ à C₄) ; un groupe alcoxy en C₁ à C₁₂, ou un groupe alcoxy en C₁ à C₄ substitué par un groupe -COO(alkyle en C₁ à C₁₈) ou -CO(OCH₂CH₂)ₙOCH₃ ; un groupe phényle, naphtyle, furyle, thiényle ou pyridyle substitué par -(OCH₂CH₂)ₙOH, -(OCH₂CH₂)ₙOCH₃, un groupe alkylthio en C₁ à C₈, phénoxy, -COO(alkyle en C₁ à C₁₈), -CO(OCH₂CH₂)ₙOCH₃, phényle ou benzoyle, dans laquelle n vaut de 1 à 20,
formules dans lesquelles
R₁₂ représente un atome d'hydrogène, un groupe alkyle en C₁ à C₈, alcényle en C₃ à C₅, phénylalkyle en C₇ à C₉, hydroxyalkyle en C₁ à C₄ ou phényle,
R₈ représente un atome d'hydrogène, un groupe alkyle en C₁ à C₈ ou phényle, et
R₉, R₁₀ et R₁₁ représentent, indépendamment les uns des autres, un atome d'hydrogène, un groupe alkyle en C₁ à C₄
ou R₉ et R₁₀ représentent conjointement un groupe alkylène en C₃ à C₇,
R₆ représente un atome d'hydrogène, un groupe alkyle en C₁ à C₁₂, un groupe alkyle en C₂ à C₄ substitué par un groupe hydroxy, alcoxy en C₁ à C₄, -CN ou -COO(alkyle en C₁ à C₄) ; un groupe alcényle en C₃ à C₅, cycloalkyle en C₅ à C₁₂ ou phénylalkyle en C₇ à C₉,
R₇ représente un groupe alkyle en C₁ à C₁₂, un groupe alkyle en C₂ à C₄ substitué par un groupe hydroxy, alcoxy en C₁ à C₄, -CN ou -COO(alkyle en C₁ à C₄) ; un groupe alcényle en C₃ à C₅, cycloalkyle en C₅ à C₁₂, phénylalkyle en C₇ à C₉, phényle ou un groupe phényle substitué par un atome d'halogène, un groupe alkyle en C₁ à C₁₂, un groupe alcoxy en C₁ à C₄ ou -COO(alkyle en C₁ à C₄), ou bien R₇ représente conjointement avec R₃ un groupe alkylène en C₁ à C₇, phénylalkylène en C₇ à C₁₀, o-xylylène, 2-buténylène ou oxa- ou aza-alkylène en C₂ à C₃, ou bien
R₆ et R₇ représentent conjointement un groupe alkylène en C₃ à C₇, qui peut être interrompu par -O-, -S-, -CO- ou -N(R₁₃)- ou substitué par un groupe hydroxy, alcoxy en C₁ à C₄ ou -COO(alkyle en C₁ à C₄), R₁₃ représentant un atome d'hydrogène, un groupe alkyle en C₁ à C₁₂, qui peut être interrompu par un ou plusieurs -O-, un groupe alcényle en C₃ à C₅, un groupe phénylalkyle en C₇ à C₉, un groupe hydroxyalkyle en C₁ à C₄, -CH₂CH₂CN, -CH₂CH₂COO(alkyle en C₁ à C₄), alcanoyle en C₂ à C₈ ou benzoyle.

3. Procédé de production de photoamorceurs de formule IV 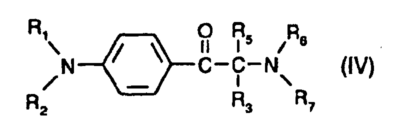 ou de leurs sels d'addition d'acide, dans laquelle :
R₁ et R₂ représentent conjointement, atome d'azote compris, un groupe morpholinyle, diméthylmorpholinyle, pipérazinyle, N-méthylpipérazinyle ou 2,5-diméthylpipérazinyle ;
R₃ représente un groupe alkyle en C₂ à C₂₀ ramifié ou non ramifié, non substitué ou substitué par un groupe alcoxy en C₁ à C₄, phénoxy, cyclohexyle ou phényle ;
R₅ représente
(a) un groupe de formule 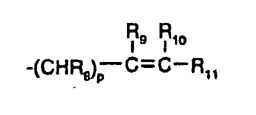 où p est égal à zéro ou 1,
ou
(b) un groupe de formule 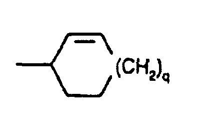 dans laquelle q est égal à 0, 1, 2 ou 3
ou
(c) un groupe de formule 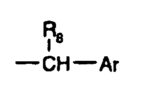
dans laquelle Ar représente un groupe alkyle en C₁ à C₁₂ non substitué ou substitué par un atome d'halogène, un groupe OH, ou un groupe alkyle en C₁ à C₄ substitué par un groupe OH, un atome d'halogène, -N(R₁₂)₂, un groupe alcoxy en C₁ à C₁₂, -COO(alkyle en C₁ à C₁₈), -CO(OCH₂CH₂)ₙOCH₃ ou -OCO(alkyle en C₁ à C₄) ; un groupe alcoxy en C₁ à C₁₂, ou un groupe alcoxy en C₁ à C₄ substitué par un groupe -COO(alkyle en C₁ à C₁₈) ou -CO(OCH₂CH₂)ₙOCH₃; un groupe phényle, naphtyle, furyle, thiényle ou pyridyle substitué par -(OCH₂CH₂)ₙOH, -(OCH₂CH₂)ₙOCH₃, un groupe alkylthio en C₁ à C₈, phénoxy, -COO(alkyle en C₁ à C₁₈), -CO(OCH₂CH₂)ₙOCH₃, phényle ou benzoyle, dans laquelle n,vaut de 1 à 20,
formules dans lesquelles
R₁₂ représente un atome d'hydrogène, un groupe alkyle en C₁ à C₈, alcényle en C₃ à C₅, phénylalkyle en C₇ à C₉, hydroxyalkyle en C₁ à C₄ ou phényle,
R₈ représente un atome d'hydrogène, un groupe alkyle en C₁ à C₈ ou phényle, et
R₉, R₁₀ et R₁₁ représentent, indépendamment les uns des autres, un atome d'hydrogène, un groupe alkyle en C₁ à C₄ ou bien R₉ et R₁₀ représentent conjointement un groupe alkylène en C₃ à C₇,
R₆ représente un atome d'hydrogène, un groupe alkyle en C₁ à C₁₂, un groupe alkyle en C₂ à C₄ substitué par un groupe hydroxy, alcoxy en C₁ à C₄, -CN ou -COO(alkyle en C₁ à C₄); un groupe alcényle en C₃ à C₅, cycloalkyle en C₅ à C₁₂ ou phénylalkyle en C₇ à C₉,
R₇ représente un groupe alkyle en C₁ à C₁₂, un groupe alkyle en C₂ à C₄ substitué par un groupe hydroxy, alcoxy en C₁ à C₄, -CN ou -COO(alkyle en C₁ à C₄) ; un groupe alcényle en C₃ à C₅, cycloalkyle en C₅ à C₁₂, phénylalkyle en C₇ à C₉, phényle ou un groupe phényle substitué par un atome d'halogène, un groupe alkyle en C₁ à C₁₂, un groupe alcoxy en C₁ à C₄ ou -COO(alkyle en C₁ à C₄), ou bien R₇ représente conjointement avec R₃ un groupe alkylène en C₁ à C₇, phénylalkylène en C₇ à C₁₀, o-xylylène, 2-buténylène ou oxa- ou aza-alkylène en C₂ à C₃, ou bien
R₆ et R₇ représentent conjointement un groupe alkylène en C₃ à C₇, qui peut être interrompu par -O-, -S-, -CO- ou -N(R₁₃)- ou substitué par un groupe hydroxy, alcoxy en C₁ à C₄ ou -COO(alkyle en C₁ à C₄), R₁₃ représentant un atome d'hydrogène, un groupe alkyle en C₁ à C₁₂, qui peut être interrompu par un ou plusieurs -O-, un groupe alcényle en C₃ à C₅, un groupe phénylalkyle en C₇ à C₉, un groupe hydroxyalkyle en C₁ à C₄, -CH₂CH₂CN, -CH₂CH₂COO(alkyle en C₁ à C₄), alcanoyle en C₂ à C₈ ou benzoyle,
au moyen de l'aminolyse d'une p-halogénophénylalkylcétone de formule II 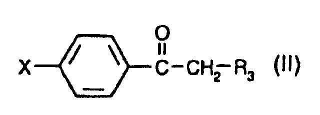 avec une amine cyclique de formule III 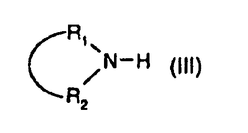 dans de l'eau, à une température d'au moins 130°C, formules dans lesquelles X représente un atome d'halogène et R₁, R₂ et R₃ ont la signification indiquée ci-dessus, pour former une phénylalkylcétone, substituée par une amine cyclique, de formule I 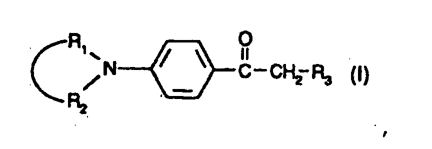 suivie de l'halogénation de ce composé phénylalkylcétone de formule I, de la réaction avec une amine de formule 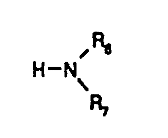 puis de la réaction avec un composé introduisant le groupe R₅ et d'une transposition de Stevens dans des conditions basiques.
